# EUROPEAN PATENT SPECIFICATION

(11) **EP 2 837 354 B1**
(45) Date of publication and mention of the grant of the patent: **16.01.2019**
(21) Application number: 13775568.2
(22) Date of filing: 11.04.2013
(51) Int. Cl.: A61B 17/00, A61B 90/00

(54) **MEDICAL MANIPULATOR**
MEDIZINISCHER MANIPULATOR
DISPOSITIF MÉDICAL DE MANIPULATION

(30) Priority: 12.04.2012 JP 2012091262
(43) Date of publication of application: 18.02.2015
(73) Proprietor: Karl Storz SE & Co. KG, 78532 Tuttlingen (DE)
(72) Inventor: ISHIDA, Shinji, Fujinomiya-shi Shizuoka 418-0015 (JP); SANO, Hiroaki, Fujinomiya-shi Shizuoka 418-0015 (JP); SUZUKI, Tsuneyoshi, Kanuma-shi Tochigi 322-0251 (JP); MUGISHIMA, Hirofumi, Kanuma-shi Tochigi 322-0251 (JP)
(74) Representative: Witte, Weller & Partner Patentanwälte mbB
(86) International application number: PCT/JP2013/060948
(87) International publication number: WO 2013/154159

(56) References cited:
- WO-A1-2012/002299
- WO-A2-2007/018898
- JP-A- 2000 229 084
- JP-A- 2004 097 766
- JP-A- 2007 502 198
- JP-A- 2009 505 688
- US-A- 5 766 196
- US-A1- 2009 198 272
- US-A1- 2010 121 138
- US-A1- 2011 184 459

## Description

### Technical Field

The present invention relates to a medical manipulator by which an end effector can be inserted into the interior of a living body for enabling a treatment to be carried out on a treatment target.

### Background Art

In a surgical treatment, without performing a thoracotomy or a laparotomy with respect to a patient, small holes are opened in the body, forceps, scissors, or the like are introduced into the body through the holes, and a desired surgical treatment (a so-called endoscopic surgical procedure) is carried out on diseased biological tissue or the like in the body. Recently, in this type of treatment, in addition to forceps or scissors, a medical manipulator is used, which is capable of performing operations with a higher degree of freedom in the living body.

For example, with the medical manipulator disclosed in Japanese Laid-Open Patent Publication No. 2011-072570, during the surgical treatment, an end effector, which is disposed on a distal end side of a shaft, is inserted into the living body. The end effector is constituted as a gripper that is capable of gripping a target object to be treated and then applying electrical energy to the target object to be treated. By transmitting an operating force from an operating element (main body portion) disposed on the proximal end side of the shaft, variations in posture (for example, a yawing operation to tilt the gripper in a direction differing from the axial direction of the shaft, a rolling operation to rotate the gripper about the axis of the shaft, etc.) or opening and closing operations of the gripper are carried out.

In the case that a surgical treatment is carried out using the medical manipulator, a plurality of holes are opened in the abdomen or the like of a patient, and an endoscope (camera) and a forceps or the like are inserted into the body cavity, together with inserting a distal end (gripper) of the medical manipulator. Consequently, under observation of the camera, the gripper is delivered to the site of a living tissue (treatment object) in the body, and by a closing operation of the gripper, the living tissue is gripped while electrical current is applied to the living tissue. During delivery of the gripper or when a treatment on the living tissue is performed, based on the operating force transmitted from the main body portion, the state of the gripper (the posture or the opening/closing condition thereof) can freely be changed at locations in the living body.
WO 2007/018898 A2 discloses an instrument comprising an elongated guide shaft having proximal and distal ends and including an instrument lumen for receiving there through a manually operated instrument having an instrument shaft. A distal bendable member is disposed at the distal end of the guide shaft and a proximal bendable member is disposed at the proximal end of the guide shaft. Actuation means extends between the distal and proximal bendable members and provides a bending of the distal bendable member controlled from the proximal bendable member. The proximal bendable member is controlled from the manually operated instrument to cause a corresponding bending of said distal bendable member.
US 5 766 196 A discloses a medical instrument with a steerable distal end, having a handle for actuating the instrument, a distal operating section for performing a medical procedure, a middle section connecting the handle with the operating section, at least a portion of the middle section being bendable. The instrument further comprises a control member for controlling the direction of movement of the bendable section in three dimensions. A protective sheath may be disposed over the bendable section.

### Summary of Invention

Claim 1 defines the invention and the dependent claims disclose the preferred embodiments. The present invention has been devised in relation to the above-described medical manipulator, and has the object of providing a medical manipulator, which enhances the operability of an end effector that carries out a treatment on a treatment target, whereby the treatment can be implemented efficiently and with improved accuracy.

To accomplish the aforementioned objects, the present invention is characterized by a medical manipulator according to claim 1, including a shaft, a distal end working unit provided on a distal end of the shaft and having an end effector configured to carry out a treatment on a treatment target, and a main body portion disposed on a proximal end of the shaft and configured to operate the end effector based on an input from a person. The distal end working unit includes a bending portion disposed between the end effector and the shaft and which causes the distal end working unit to bend in a direction that differs from an axial direction of the shaft, in accordance with an operating force in the bending direction that is transmitted from the main body portion, and a tube, which is disposed at a position overlapping with the bending portion and is capable of bending in following relation to the movement of the bending portion. The tube transmits a rotational operating force, which is transmitted from the main body portion, to the end effector, so as to enable the end effector to be rotated through an unlimited range of rotation.

According to the above description, the tube that is disposed in a position overlapping with the bending portion bends in following relation with the movement of the bending portion. Therefore, for example, even in the case that the treatment target is located deeply inside the living body, the posture of the end effector can easily be changed, and the end effector can be delivered smoothly to the treatment target. Further, the tube delivers the operating force in the rotational direction to the end effector, and the end effector is capable of rotating through an unlimited range of rotation, whereby the posture of the end effector at the distal end side of the bending portion can freely be changed, and a precise treatment can be carried out by changing the orientation of the end effector any number of times to conform with the treatment target. More specifically, high operability for the end effector is obtained, and the procedure performed on the treatment target can be conducted efficiently and accurately.

The bending portion may include a structure in which three or more rigid joint members are arrayed in the axial direction, and the adjacent joint members are connected to each other so as to bend mutually. Further, the tube may extend over a length that is longer than the axial length of the bending portion.

In this manner, with a structure in which three or more joints are subjected to bending, the bending portion can be bent gently and gradually. Further, the tube, which extends a greater length than the bending portion, easily bends in following relation to the bending portion, so that even in a bent state, the operating force in the direction of rotation can be transferred smoothly to the end effector.

The distal end working unit transmits operating forces in a distal end direction and a proximal end direction, which are transferred from the main body portion, to the end effector through the tube.

In this manner, by transferring operating forces in the distal end and proximal end directions, to the end effector through the tube, the end effector can be caused to carry out various operations by the operating forces in the distal end and proximal end directions.

In this case, the end effector is a gripper in which first and second gripper members are opened and closed to grip the treatment target. The gripper can close the first and second gripper members by the operating force in the proximal end direction that is transmitted through the tube, and can open the first and second gripper members by the operating force in the distal end direction that is transmitted through the tube.

In this manner, assuming a structure is provided in which the first and second gripper members are opened and closed by operating forces in the distal end and proximal end directions, the treatment target can easily be sandwiched and gripped between the gripper members, and a predetermined treatment can be performed efficiently.

Furthermore, the distal end working unit may include an outer shell member connected to a distal end of the bending portion, and a retaining member, which retains the end effector under a condition in which at least a portion of the retaining member is accommodated in the interior of the outer shell member, and is rotated relatively with respect to the outer shell member by the rotational operating force from the tube.

Consequently, since the tube and the retaining member can be rotated relatively with respect to the bending portion and the outer shell member, the end effector that is held on the retaining member can be rotated suitably.

In this case, the outer shell member may include a groove that is engraved in a circumferential direction on an inner wall confronting an outer surface of the retaining member, and the retaining member may include a projection that is inserted rotatably in the groove.

In this manner, by inserting the projection of the retaining member rotatably in the groove, rotation of the retaining member is guided by the groove, and therefore, the retaining member (i.e., the end effector) can be rotated more reliably.

Further, plural coils may be disposed in the interior of the tube in an overlapping manner so as to surround a hollow portion that extends in the axial direction, and at least two coils among the plural coils may be wound in mutually different winding directions.

In this manner, by arranging the two coils, which are wound in different winding directions, in the interior of the tube, the strength of the tube can be increased. As a result, the rotational operating forces from the shaft can be transferred smoothly to the end effector through the tube.

According to the present invention, the operability of an end effector that carries out a treatment on a treatment target can be improved, whereby the treatment can be implemented efficiently and with improved accuracy.

### Brief Description of Drawings

FIG. 1 is a perspective view showing the overall structure of a medical manipulator according to an embodiment of the present invention;
FIG. 2 is a perspective view showing an enlarged representation of main components of a distal end working unit of the manipulator of FIG. 1;
FIG. 3A is a first schematic view for describing in outline an energizing operation of the manipulator of FIG. 1;
FIG. 3B is a second schematic view for describing in outline the energizing operation of the manipulator of FIG. 1;
FIG. 4A is a first schematic view for describing in outline a yawing operation of the manipulator of FIG. 1;
FIG. 4B is a second schematic view for describing in outline the yawing operation of the manipulator of FIG. 1;
FIG. 5 is a schematic view for describing in outline a rolling operation of the manipulator of FIG. 1;
FIG. 6A is a first schematic view for describing in outline an opening and closing operation of the manipulator of FIG. 1;
FIG. 6B is a second schematic view for describing in outline the opening and closing operation of the manipulator of FIG. 1;
FIG. 7 is a partial exploded perspective view showing an enlarged representation of a distal end side of the manipulator of FIG. 1;
FIG. 8 is a partial exploded perspective view showing an enlarged representation of a bending portion of the manipulator of FIG. 1;
FIG. 9 is a partial exploded perspective view showing an enlarged representation of a handle of the manipulator of FIG. 1;
FIG. 10 is a schematic plan view for describing the yawing operation of the manipulator of FIG. 1;
FIG. 11 is a partial perspective view for describing the yawing operation of the manipulator of FIG. 1;
FIG. 12 is a partial perspective view for describing an assembled condition in the interior of the handle of FIG. 1;
FIG. 13A is a side view showing main components of the bending portion of the manipulator of FIG. 1;
FIG. 13B is a plan view showing main components of the bending portion of the manipulator;
FIG. 13C is a cross sectional view taken along line XIIIC-XIIIC of FIG. 13B;
FIG. 14A is a partial perspective view showing at an enlarged scale a joint member of FIG. 13A;
FIG. 14B is a partial perspective view showing at an enlarged scale an assembled condition of the joint members;
FIG. 15 is a schematic side view for describing the rolling operation of the manipulator of FIG. 1;
FIG. 16 is a partial perspective view for describing an assembled condition in the interior of the handle of FIG. 1;
FIG. 17 is a perspective view, partially cut away, of main components of a distal end part of the manipulator of FIG. 1;
FIG. 18A is a cross sectional plan view showing a distal end part of the manipulator of FIG. 1;
FIG. 18B is a cross sectional side view showing a distal end part of the manipulator of FIG. 1;
FIG. 19 is a schematic perspective view for describing an opening and closing operation of the gripper of FIG. 1;
FIG. 20A is a first side view for describing operations on the side of the handle of FIG. 19;
FIG. 20B is a second side view for describing operations on the side of the handle of FIG. 19;
FIG. 21A is a principal plan view showing the gripper of FIG. 1;
FIG. 21B is a partial plan view showing an open state of the gripper; and
FIG. 21C is a partial plan view showing a closed state of the gripper.

### Description of Embodiments

Hereinafter, a preferred embodiment of a medical manipulator according to the present invention will be described in detail below with reference to the accompanying drawings.

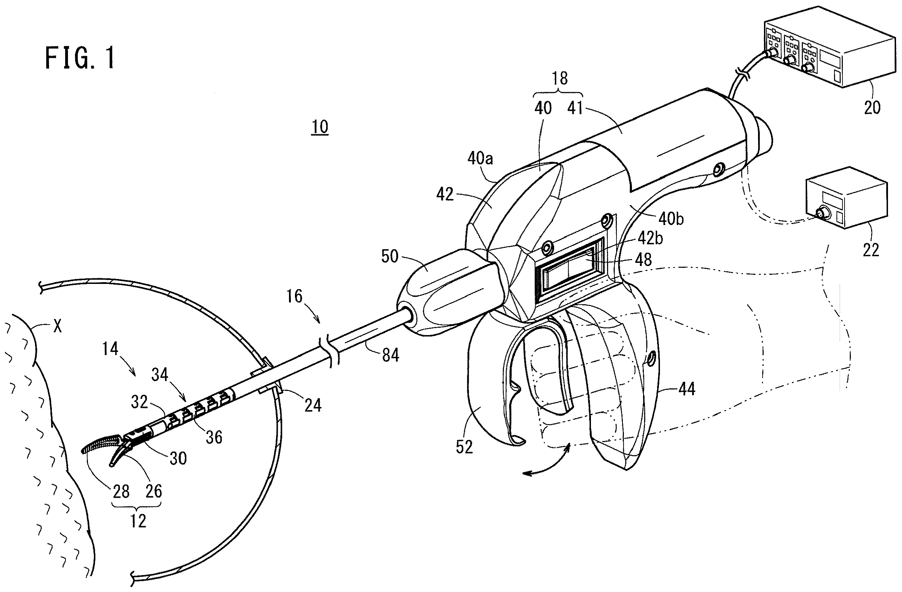

FIG. 1 is a perspective view showing the overall structure of the medical manipulator according to the present embodiment. The medical manipulator (hereinafter referred to simply as a "manipulator 10") according to the present embodiment is used in an endoscopic surgical procedure, and is configured to carry out a predetermined process (e.g., cauterization by application of heat) by applying electrical energy to a living tissue that serves as a treatment target X. As a biological tissue that serves as the treatment target X on which the manipulator 10 performs a treatment, for example, tumors (lesions), muscles, blood vessels, or nerves, etc., may be cited.

The end effector of the manipulator 10 is constituted as a gripper 12 (electrosurgical knife) that applies electrical energy to the treatment target X by gripping the treatment target X. In the interior of the manipulator 10, various mechanisms are provided for implementing delivery and gripping (an energizing operation) of the gripper 12. Below, a detailed description will be given concerning the manipulator 10.

As shown in FIG. 1, the manipulator 10 comprises a distal end working unit 14 having a gripper 12 for carrying out a surgical procedure on the treatment target X, a shaft 16 connected to a proximal end side of the distal end working unit 14 and which extends in the direction of the proximal end, and a handle 18 (main body portion) disposed on the proximal end side of the shaft 16, and which operates the distal end working unit 14 based on an operation (input) from a person or user of the manipulator 10. Further, a controller 20 is connected to the handle 18 of the manipulator 10 for supplying desired electrical power when the distal end working unit 14 is operated. In addition, a high frequency power source 22 for energizing (supplying high frequency voltage) to the gripper 12 is connected to the handle 18.

Upon use of the manipulator 10, the handle 18 is gripped and operated by the user (an operator such as a doctor or the like), and a distal end side of the manipulator 10 (the distal end working unit 14 and the distal end side of the shaft 16) is inserted into the body of a patient. At this time, the user opens a small diameter hole at a predetermined position on the body surface of the patient, installs a trocar 24 together with injecting a carbon dioxide gas, and inserts the shaft 16 into the patient via the trocar 24. Further, in a state in which the distal end side of the manipulator 10 is inserted into the body, while observations are made through an endoscope, variations in posture and opening and closing operations of the gripper 12 are carried out appropriately. Consequently, the gripper 12 is delivered to the site of the treatment target X, and a procedure is performed to supply electrical current to the treatment target X.

The structure of the end effector is not limited to a gripper 12 that supplies current to the treatment target X, and various alternative structures may be adopted for the end effector. For example, as the end effector, there may be applied a scissors or a surgical knife (blade) for cutting living tissue. Further, the end effector may be constituted as a gripping device for gripping a medical device (forceps, needle, etc.), and a surgical procedure can be performed on the treatment target X by the medical device that is gripped.

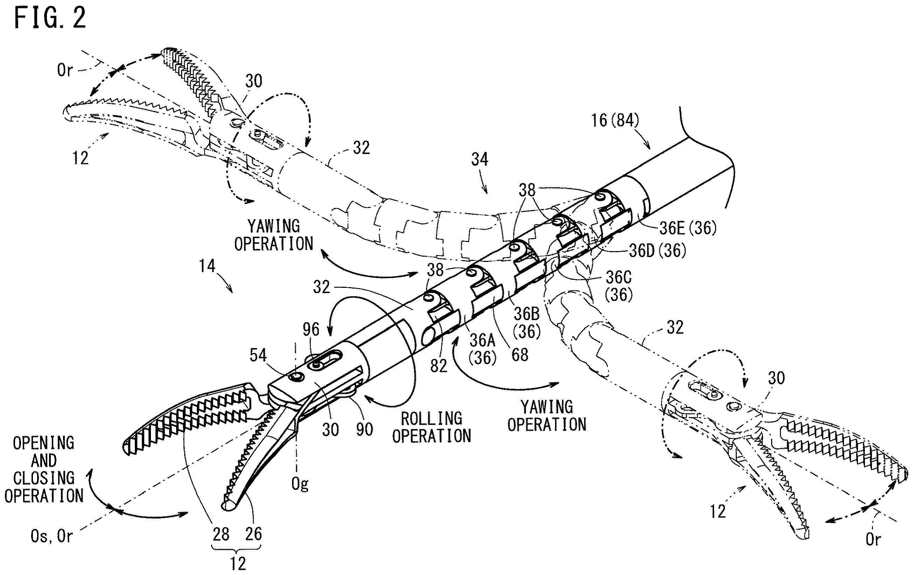

As shown in FIG. 2, variations in posture and opening and closing operations of the gripper 12 are implemented on a distal end working unit 14 on the distal end of the manipulator 10. As changes in the posture of the gripper 12, there may be cited a yawing operation by which the gripper 12 is tilted so as to curve in a lateral direction (yawing direction) with respect to an axis Os of the shaft 16, and a rolling operation in which the gripper 12 is rotated about an axis Or of the gripper 12. Naturally, the variations in posture of the gripper 12 are not limited to a yawing operation and a rolling operation. For example, a pitch operation may be performed in which the gripper 12 is tilted in a vertical direction (pitch direction) with respect to the axis Os of the shaft 16.

The gripper 12 is constituted from two gripper members (first gripper member 26, second gripper member 28), which are held while being capable of opening and closing by a gripper retaining member 30. Distal end parts of the first and second gripper members 26, 28 are subjected to an opening and closing operation about a vertically oriented axis Og of the gripper retaining member 30. The proximal end side of the gripper retaining member 30 is inserted rotatably through a cylindrically shaped outer shell member 32. A bending portion 34 (joint part) that carries out the yawing operation of the distal end working unit 14 is connected to the proximal end side of the outer shell member 32.

The bending portion 34 is constituted by juxtaposing in the axial direction a plurality of joint members 36 (five are shown in FIG. 2), which are made of a hard material, for example, stainless steel (hereinbelow, a description will be given in which the letters A through E are appended to each of the respective joint members 36 in order from the distal end side). The adjacent joint members 36 are connected so as to be mutually rotatable about hinge members 38. Further, concerning the joint members 36, the joint member 36A on the furthest most distal end side is connected in a bendable fashion to the outer shell member 32, whereas the joint member 36E on the furthest most proximal end side is connected and fixed to the distal end of the shaft 16. In the present embodiment, although stainless steel is used as the material for the joint members 36, the invention is not limited to this feature, insofar as the joint members 36 are excellent in durability and are capable of realizing the functions of the present invention. For example, a polyether ether ketone resin (PEEK) may be used.

The yawing operation of the distal end working unit 14 is implemented by continuing to bend in a linked manner the five hinge members 38 about the operational fulcrum of the joint member 36E. Stated otherwise, by the outer shell member 32 and the joint members 36A through 36D being inclined in a yawing direction at substantially the same angle, the gripper 12 and the gripper retaining member 30 on the distal end side of the bending portion 34 are tilted integrally. The movable range of the yawing operation of the bending portion 34 can be set appropriately. For example, if the movable range is set up to a range at which the roll axis Or of the gripper 12 becomes perpendicular to the axis Os of the shaft 16, i.e., a range in which the gripper 12 can be tilted to the left and right, that is, tilted 180°, the facing orientation of the gripper 12 can be varied over a wide range within the body.

On the other hand, the rolling operation of the distal end working unit 14 is carried out by rotating the gripper retaining member 30 relatively to the outer shell member 32. Stated otherwise, the outer shell member 32 that is connected to the bending portion 34 is maintained in a fixed condition with respect to the direction of rotation, and the gripper retaining member 30 rotates with respect to the outer shell member 32. Therefore, the gripper 12 and the gripper retaining member 30 rotate together in unison. Further, in the present embodiment, the movable range (range of rotation of the gripper 12) of the rolling operation is unlimited.

Furthermore, the opening and closing operation of the gripper 12 is carried out by opening and closing the first and second gripper members 26, 28, i.e., by having the distal end parts of the first and second gripper members 26, 28 approach toward (abut) and separate away from each other, in a state of being held by the gripper retaining member 30. The opening and closing operation of the gripper 12 can be realized at a desired timing based on operations of the user, regardless of any change in the posture of the gripper 12.

In the present embodiment, the variation in posture (the yawing operation and the rolling operation) of the gripper 12 and the opening and closing operation of the gripper 12 are carried out based on operating forces that are transmitted from the shaft 16 to the distal end working unit 14.

Returning to FIG. 1, the shaft 16 that is connected to the distal end working unit 14 extends in a straight line over a predetermined length (e.g., 350 mm), and the handle 18 is connected to a proximal end portion of the shaft 16. The shaft 16 includes a function to transfer operating forces from the handle 18 to the distal end working unit 14. Further, during a surgical procedure, the proximal end side of the shaft 16 is exposed outside the body of the patient, and by manipulating the position and angle of the manipulator 10 (shaft 16) from the outside, the insertion angle and insertion amount of the distal end working unit 14 that is inserted in the body are changed.

The handle 18 includes a handle main body 40 that is formed in the shape of a pistol for enabling easy gripping thereof by one hand of the user, and a drive unit 41 that is capable of being attached to and detached from an upper proximal end side portion of the handle main body 40. The handle main body 40 includes a body portion 42, which extends in the same direction as the axial direction of the shaft 16, and a gripping unit 44 that extends downwardly from a lower side of the body portion 42.

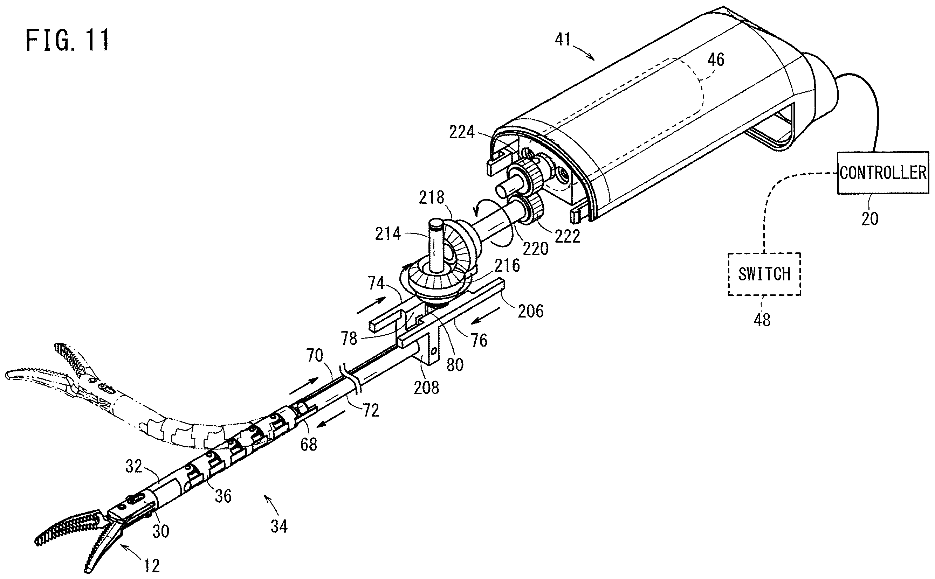

The body portion 42 includes an internal space (not shown) having a comparatively large volume, and is provided with various mechanisms in the internal space for effecting operations (the yawing operation, the rolling operation, the opening and closing operation of the gripper 12) of the above-described distal end working unit 14. On the other hand, a drive motor 46 (see FIG. 11) that serves as a drive source for the yawing operation of the distal end working unit 14 is accommodated in the interior of the drive unit 41. Further, the aforementioned controller 20 and the high frequency power source 22 are connected to the drive unit 41. The controller 20 is connected to a non-illustrated power source, and includes a function to control rotary driving of the drive motor 46. The high frequency power source 22 includes a function to supply power (high frequency voltage) to the gripper 12 based on manipulations performed by the user.

Switches 48 (tilting operation unit) that carry out the yawing operation of the distal end working unit 14 are connected to side surfaces of the body portion 42. Further, a rotating handle 50 (rolling operation unit) that carries out the rolling operation of the distal end working unit 14 is disposed in surrounding relation to an outer side of the shaft 16 at a portion where the shaft 16 is connected to the distal end side of the body portion 42. Furthermore, a trigger 52 (opening and closing operation unit) that carries out the opening and closing operation of the gripper 12 is disposed in front of the gripping unit 44 on a lower side of the body portion 42.

The gripping unit 44 is formed somewhat more toward the distal end side than a middle region in the axial direction of the body portion 42, and has a structure such that, in a condition in which a user grips the gripping unit 44 with one hand, the switches 48, the rotating handle 50, and the trigger 52 can be operated by a finger of the one hand that has gripped the gripping unit 44.

Operations of the distal end working unit 14 are carried out by the switches 48, the rotating handle 50 and the trigger 52 being manipulated suitably by the user. The manipulator 10 according to the present embodiment performs a treatment on the treatment target X by mutually linking changes in posture (the yawing operation, the rolling operation) and the opening and closing operation of the gripper 12, and an energizing operation based on the opening and closing operation of the gripper 12. In the following description, to facilitate understanding of the invention, structures related to such operations will be described separately below in outline.

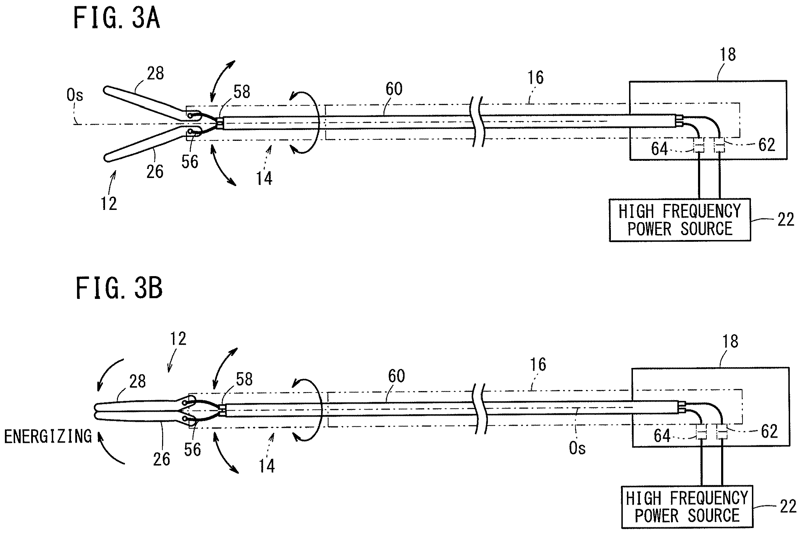

FIG. 3A is a first schematic view for describing in outline the energizing operation of the manipulator 10 of FIG. 1, and FIG. 3B is a second schematic view for describing in outline the energizing operation of the manipulator 10 of FIG. 1. First, a structure for enabling the energizing operation of the manipulator 10 will be described in outline.

As described above, the manipulator 10 includes a function to supply electrical current to the treatment target X while the treatment target X is gripped by the gripper 12. Therefore, the first and second gripper members 26, 28 are made from a metal material, and constitute electrodes (a minus electrode and a plus electrode) that supply current to the treatment target X. More specifically, the gripper 12 according to the present embodiment is a bipolar type of electrosurgical knife. Naturally, the invention is not limited to such a structure, and the end effector may be constituted as a monopolar type of electrosurgical knife.

The first gripper member 26 and the second gripper member 28 are pivotally supported in an insulated state by a single fulcrum pin 54 (see FIG. 2). When the operating force of the opening and closing operation is received, the distal end parts of the first and second gripper members 26, 28 are made to approach and separate away from one another about the fulcrum pin 54. Therefore, although supply of current is interrupted in an open state in which the distal end parts of the first and second gripper members 26, 28 are separated, in a closed state in which the distal end parts of the first and second gripper members 26, 28 are brought into abutment (including a condition of an indirectly closed state upon sandwiching the treatment target X), the first and second gripper members 26, 28 become energized to thereby supply electrical current to the treatment target X.

Respective metal members 56 (conductive paths) are connected electrically to the proximal end sides of the first and second gripper members 26, 28. The metal members 56 (e.g., copper wires) of two polarities are covered by insulating members 58 at portions thereof that extend along the bending portion 34 and the shaft 16, and the two insulating members 58 extend in the form of a single combined conductive line 60 as a result of being joined by welding or an adhesive. The conductive line 60 extends toward the proximal end together with the shaft 16, and is inserted into the handle 18. In the present embodiment, the conductive line 60 is arranged to pass through the axial center (axis Os) of the bending portion 34 and the shaft 16. Consequently, since there is no need to provide separate wiring for the conductive line on the side surface of the distal end working unit 14 or the shaft 16, entanglement of the conductive line by operating the distal end working unit 14 is avoided, and supply of current can suitably be performed.

The conductive line 60 extends to the proximal end side of the shaft 16 that is inserted into the interior of the handle 18, and the two metal members 56 in the conductive line 60 are connected respectively to a pair of cylindrical terminals 62 (rotating terminals) that are disposed on the shaft 16. Contact terminals 64 are in contact respectively with the pair of cylindrical terminals 62, and the contact terminals 64 are connected to the high frequency power source 22 externally of the handle 18.

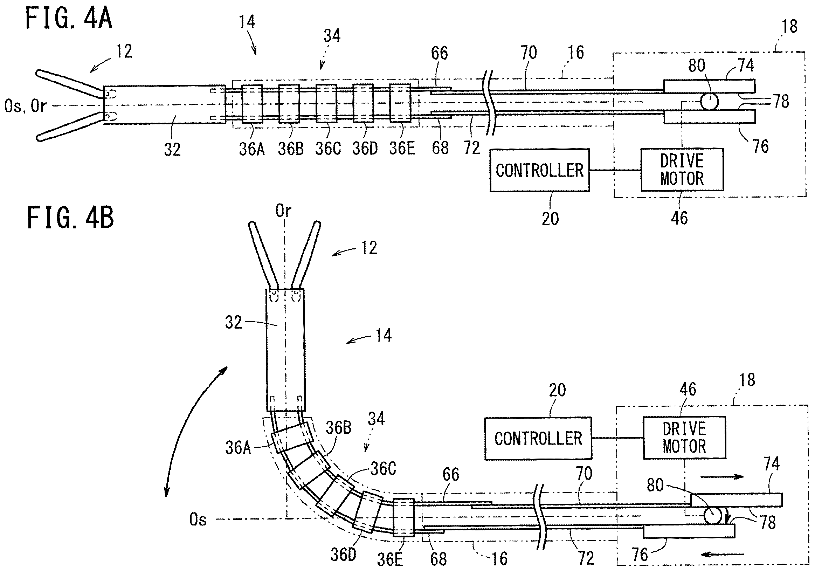

FIG. 4A is a first schematic view for describing in outline the yawing operation of the manipulator 10 of FIG. 1, and FIG. 4B is a second schematic view for describing in outline the yawing operation of the manipulator 10 of FIG. 1. Next, a structure for implementing the yawing operation will be described in outline.

As described above, the yawing operation of the manipulator 10 is realized by the bending portion 34 that is connected to the distal end working unit 14. On inner side surfaces in the width direction of the five joint members 36 that are arrayed in the axial direction, a pair of belts (a first belt 66 and a second belt 68) are inserted. The respective joint members 36 hold the first and second belts 66, 68 slidably. The distal end sides of the first and second belts 66, 68 are connected to the outer shell member 32, and the proximal end sides thereof are connected to a pair of semicircular pipes (first semicircular pipe 70, second semicircular pipe 72: bending operation force transmitting members) that extend inside the shaft 16.

The first and second semicircular pipes 70, 72 are inserted through the interior of the shaft 16 extending to the handle 18, and are connected to a pair of slide members (first slide member 74, second slide member 76), which are disposed in the interior of the handle 18. The first and second slide members 74, 76 are arranged at mutually confronting positions, with racks 78 being formed respectively on confronting surfaces thereof. Further, between the first and second slide members 74, 76, a single pinion 80 (rotating member) is arranged in meshing relation with both of the racks 78. The pinion 80 is connected mechanically to the drive motor 46 through a plurality of gears, which will be described later.

With the manipulator 10, which is constructed in this manner, for example, as shown in FIG. 4B, when the pinion 80 is rotated clockwise by rotary driving of the drive motor 46, the first slide member 74 undergoes sliding movement in the proximal end direction, and the second slide member 76 undergoes sliding movement in the distal end direction. Therefore, the first semicircular pipe 70, which is connected to the first slide member 74, also undergoes sliding movement in the proximal end direction, and the second semicircular pipe 72, which is connected to the second slide member 76, also undergoes sliding movement in the distal end direction. The operating forces of the first and second semicircular pipes 70, 72 are transmitted to the distal end working unit 14 (bending portion 34).

More specifically, by the first belt 66 being moved relatively to the bending portion 34 in the proximal end direction, the first belt 66 in the bending portion 34 becomes shorter, and by the second belt 68 being moved relatively to the bending portion 34 in the distal end direction, the second belt 68 in the bending portion 34 becomes longer. As a result, the five joint members 36 tilt in conjunction to the side of the shortened first belt 66, the outer shell member 32 that is connected to the distal end side of the joint members 36 is tilted, and the gripper 12 and the gripper retaining member 30 are made to move laterally (in the yawing direction) with respect to the axis Os of the shaft 16.

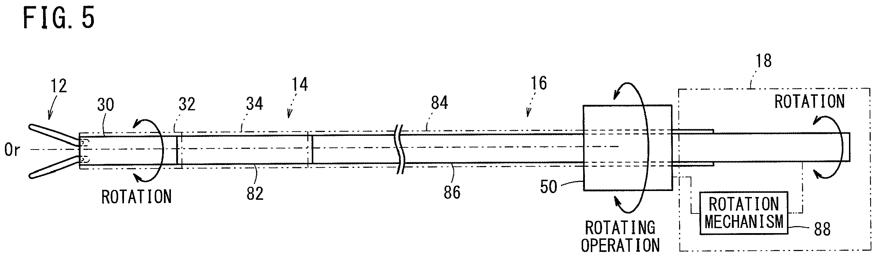

FIG. 5 is a schematic view for describing in outline the rolling operation of the manipulator 10 of FIG. 1. Next, a structure for implementing the rolling operation will be described in outline.

As described above, the rolling operation of the manipulator 10 is carried out by rotating the gripper 12 and the gripper retaining member 30 relatively with respect to the outer shell member 32. A rotatable hollow tube 82 is connected to the proximal end side of the gripper retaining member 30, and the hollow tube 82 is inserted through the interior of the bending portion 34 (the five joint members 36). The hollow tube 82 is flexible and is capable of following the bending movement of the bending portion 34, and is constructed as a torque tube, which is capable of rotating even if the hollow tube 82 is bent in following relation to the bending portion 34. The conductive line 60 (see FIG. 3A) is inserted through the interior of the hollow tube 82. The outer diameter of the hollow tube 82 preferably is 2.5 mm to 3.0 mm, and in the present embodiment, a description is given in which the outer diameter thereof is 2.7 mm.

The shaft 16 that is connected to the bending portion 34 is constituted by a double tube structure, including an outer tube 84 that makes up the exterior and extends in the axial direction, and an inner tube 86 that is inserted through the interior of the outer tube 84. The hollow tube 82 is connected to the inner tube 86 of the shaft 16, and rotates by receiving an operating force (rotational torque) in the direction of rotation that is transmitted from the inner tube 86.

The proximal end side of the outer tube 84 is inserted into the handle 18, and is connected fixedly at a distal end position of the handle 18. On the other hand, the inner tube 86 extends toward the proximal end more so than the outer tube 84, and is supported rotatably by a rotation mechanism 88 in the interior of the handle 18. The rotation mechanism 88 includes a plurality of gears, and the inner tube 86 is connected mechanically through the plural gears to the rotating handle 50 that is disposed on the distal end side of the handle 18. The rotating handle 50 is rotatable along the circumferential direction of the outer tube 84.

Accordingly, when the user manually performs a rotating operation on the rotating handle 50, the rotational torque of the rotating handle 50 is transmitted to the interior of the handle 18 on the proximal end side, and the inner tube 86 is made to rotate through the rotation mechanism 88. The operating force in the direction of rotation of the inner tube 86 is transmitted to the hollow tube 82, and the hollow tube 82 rotates together with the inner tube 86. As a result, the gripper retaining member 30 (gripper 12), which is connected to the distal end side of the hollow tube 82, is rotated about the axis Or of the gripper 12. In particular, in the manipulator 10 according to the present embodiment, the gripper 12, the gripper retaining member 30, the hollow tube 82, and the inner tube 86 are arranged in a separated state with respect to the outer shell member 32, the bending portion 34, and the outer tube 84, and are capable of being rotated through an unlimited range of rotation. Accordingly, a change in posture of the gripper 12 by the rolling operation can be carried out any number of times.

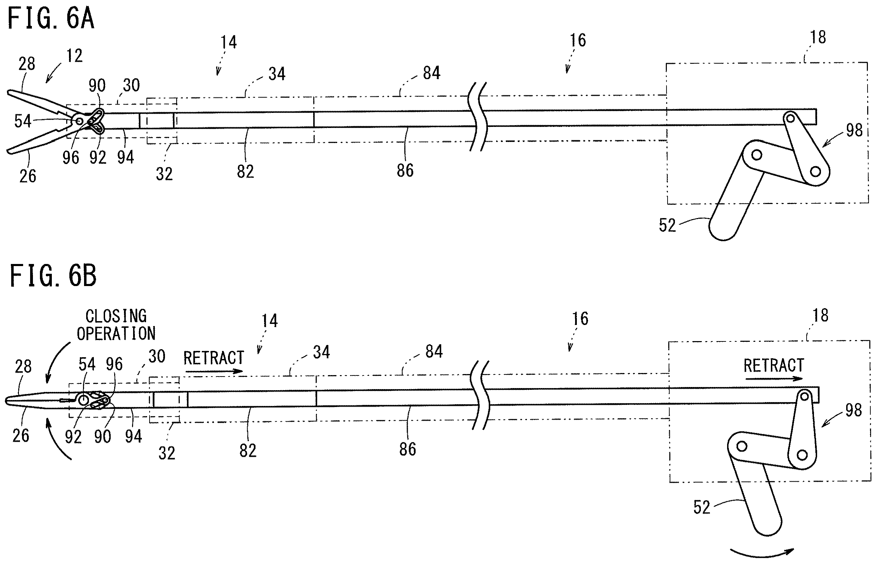

FIG. 6A is a first schematic view for describing in outline the opening and closing operation of the manipulator 10 of FIG. 1, and FIG. 6B is a second schematic view for describing in outline an opening and closing operation of the manipulator 10 of FIG. 1. Next, a structure for implementing the opening and closing operation will be described in outline.

The opening and closing operation of the gripper 12 is realized by transmission of the operating force in the distal end and proximal end directions from the handle 18 with respect to the gripper 12. The first and second gripper members 26, 28 comprise extension pieces 90 that extend backward obliquely toward the proximal end side beyond the fulcrum pin 54 about which the first and second gripper members 26, 28 pivot mutually. Long holes 92 are formed in the extension pieces 90. A moving body 94, which is capable of advancing and retracting (i.e., moving in the distal end and proximal end directions) relatively with respect to the gripper retaining member 30, is arranged in the interior of the gripper retaining member 30. A movable pin 96, which is attached to the moving body 94, is inserted into the long holes 92. The relationship in which the gripper retaining member 30 and the moving body 94 are arranged is a relationship where, during the rolling operation, the gripper retaining member 30 and the moving body 94 move together, however, during the opening and closing operation, the gripper retaining member 30 does not move, and the moving body 94 makes advancing and retracting movements. Therefore, the movable pin 96 (moving body 94) approaches toward and separates away from the fulcrum pin 54 of the gripper retaining member 30.

The above-described hollow tube 82 is connected to the proximal end side of the moving body 94. The moving body 94 undergoes advancing and retracting movements by transmission of the operating force in the distal end and proximal end directions from the hollow tube 82. Further, the above-described inner tube 86 is connected to the proximal end side of the hollow tube 82, and an advancing and retracting movement mechanism 98 in the handle 18 is connected to the proximal end side of the inner tube 86. The advancing and retracting movement mechanism 98 includes a plurality of links, which mechanically connect the inner tube 86 with the trigger 52 that is disposed on the lower side of the handle 18.

For example, when the user performs a manual operation to pull the trigger 52, the operating force of the trigger 52 is transmitted to the proximal end side within the handle 18, and the inner tube 86 is moved in the direction of the proximal end through the advancing and retracting movement mechanism 98. The operating force in the proximal end direction of the inner tube 86 is transmitted to the moving body 94 through the hollow tube 82, whereby the moving body 94 undergoes a retracting movement. Conversely, if a pressing operation of the trigger 52 is performed, the moving body 94 undergoes an advancing movement.

As shown in FIG. 6A, in the position at which the moving body 94 is moved forward (i.e., in a state in which the movable pin 96 is located on the distal end side of the long holes 92), the extension pieces 90 of the first and second gripper members 26, 28 intersect at the location of the movable pin 96. Therefore, the distal end portions of the first and second gripper members 26, 28 separate away from one another and are placed in an open condition. As shown in FIG. 6B, at the position at which the moving body 94 is retracted (i.e., in a state in which the movable pin 96 is located on the proximal end side of the long holes 92), the long holes 92 of the first and second gripper members 26, 28 are guided, and the respective extension pieces 90 come into mutual proximity (overlap) with each other. Accordingly, the distal end portions of the first and second gripper members 26, 28 approach one another mutually and are placed in a closed condition. In this manner, at the gripper 12, an opening and closing operation is realized by transmission of the operating force in the distal end and proximal end directions from the hollow tube 82.

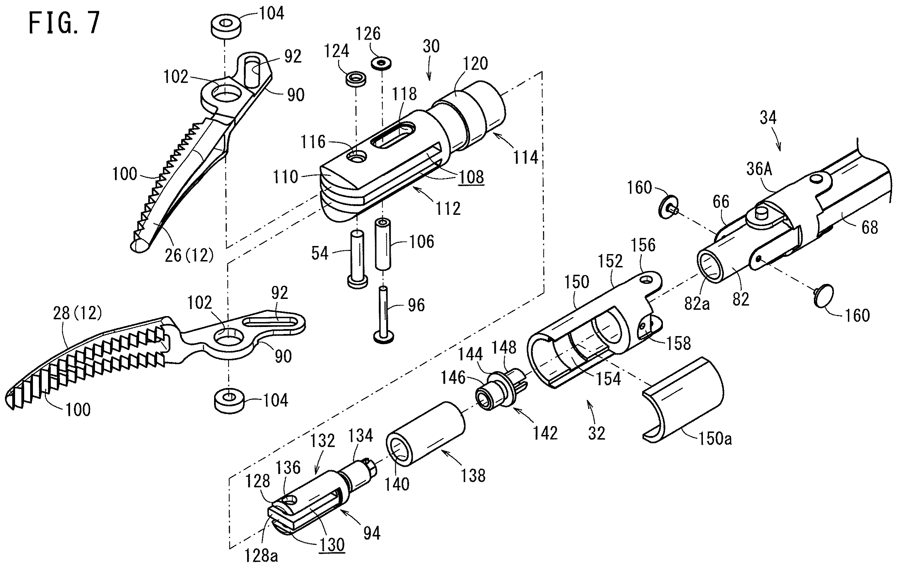

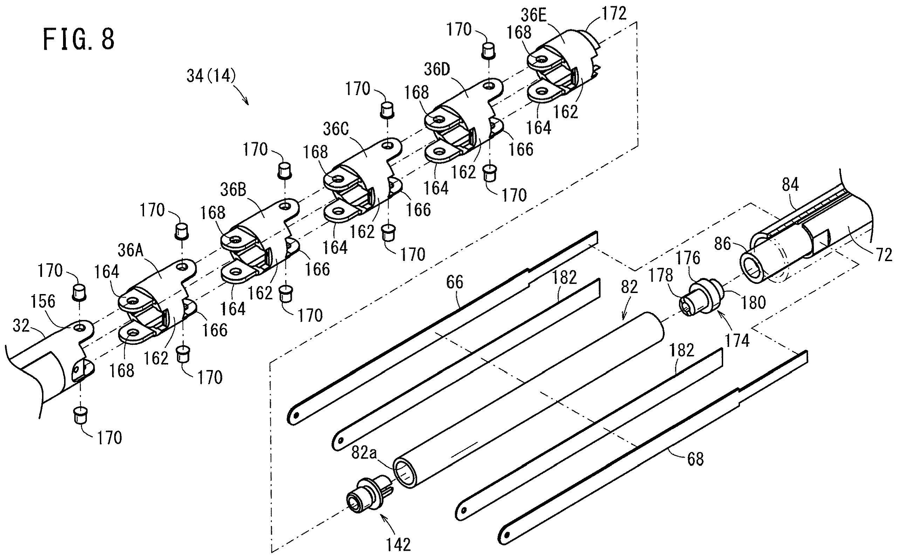

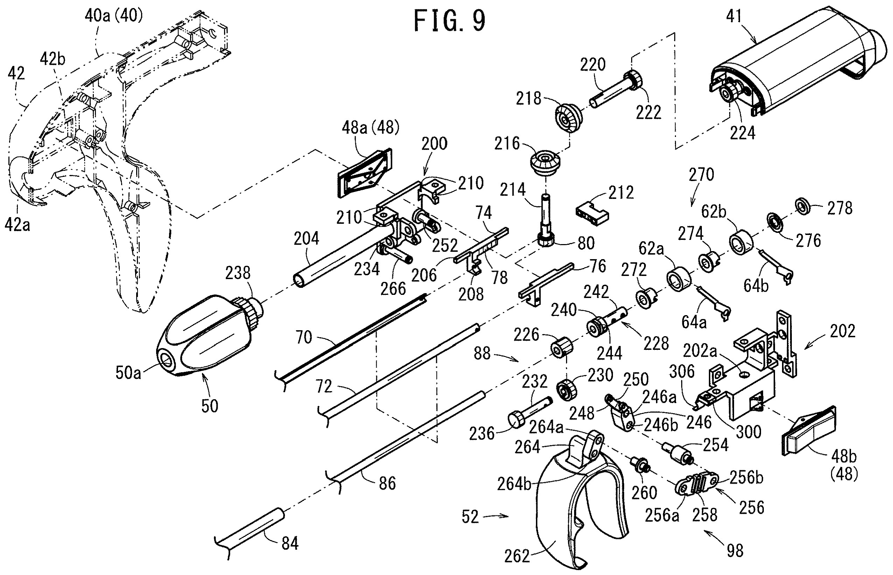

FIG. 7 is a partial exploded perspective view showing an enlarged representation of a distal end side of the manipulator 10 of FIG. 1, FIG. 8 is a partial exploded perspective view showing an enlarged representation of the bending portion 34 of the manipulator 10 of FIG. 1, and FIG. 9 is a partial exploded perspective view showing an enlarged representation of the handle 18 of the manipulator 10 of FIG. 1. Next, with reference to FIGS. 7 through 9, members that make up the manipulator 10 according to the present embodiment will be described in detail.

The first and second gripper members 26, 28 that constitute the gripper 12 are of a shape that extends in the distal end direction while the distal end portions are curved downward, and serrated meshing teeth 100 are formed on mutually confronting surfaces thereof. The meshing teeth 100 of the first and second gripper members 26, 28 enmesh with one another in a closed state. Further, the extension pieces 90 of the first and second gripper members 26, 28 are formed in flat plate shapes having a predetermined plate thickness (e.g., 0.5 mm), which extend from proximal end portions of the meshing teeth 100. Round holes 102 in which the fulcrum pin 54 is fitted are bored through the extension pieces 90 at portions near the distal ends thereof. The fulcrum pin 54 is supported by insulating rings 104, which are fitted into the round holes 102. Long holes 92 are bored through proximal end sides of the extension pieces 90. A movable pin 96 is inserted through an insulating tube 106 in the long holes 92. Further, the extension pieces 90 of the first and second gripper members 26, 28 are inserted respectively into gaps 108 on the distal end side of the gripper retaining member 30.

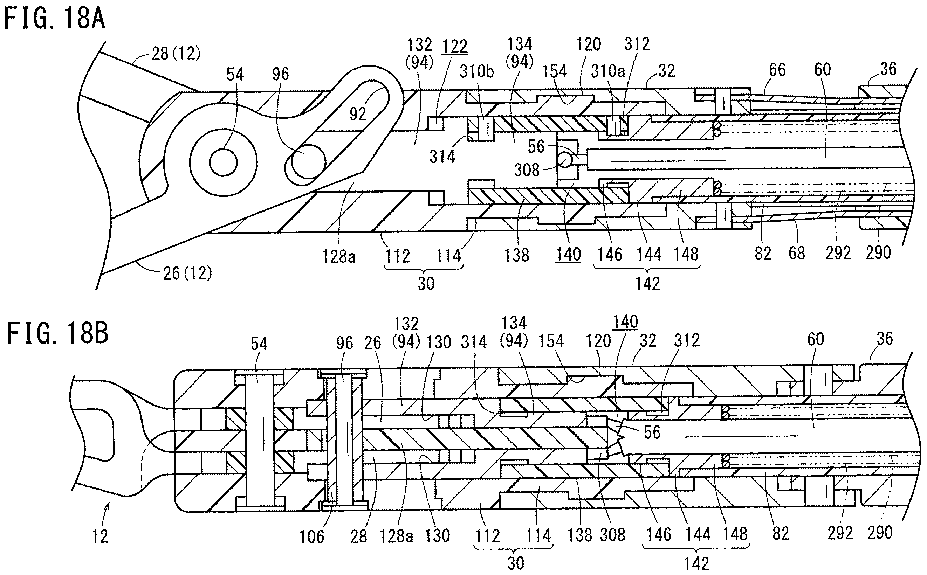

The gripper retaining member 30 is made up from a three-pronged retaining member side section 112 that extends toward the distal end such that plate-shaped retaining plates 110 branch in three prongs so as to form the gaps 108, and an engagement tubular portion 114, which extends toward the proximal end and joins with the proximal end side of the three-pronged retaining member side section 112. Fulcrum pin holes 116 through which the fulcrum pin 54 is inserted are formed on distal end parts of the three retaining plates 110, and movable pin long holes 118 through which the movable pin 96 is inserted are formed on a proximal end side of the fulcrum pin holes 116. A projection 120, which projects radially outward and extends in the circumferential direction, is disposed on an outer surface of the engagement tubular portion 114. Further, within the three-pronged retaining member side section 112 and the engagement tubular portion 114, a sliding space 122 through which the moving body 94 is inserted (see FIG. 18A) is formed in a continuous manner.

In a condition in which the first and second gripper members 26, 28 are arranged in the gaps 108, the fulcrum pin 54 is inserted through the fulcrum pin holes 116, and the end of the fulcrum pin 54 is fixed by a washer 124, whereby the first and second gripper members 26, 28 are pivotally supported. In a condition in which the first and second gripper members 26, 28 are fixed by the fulcrum pin 54, and the moving body 94 has been inserted through the sliding space 122, the movable pin 96 is inserted through the long holes 92 and the movable pin long holes 118, and by fixing the end of the movable pin 96 through a washer 126, the movable pin 96 is arranged so as to be capable of advancing and retracting with respect to the long holes 92 and the movable pin long holes 118.

Similar to the gripper retaining member 30, the moving body 94 has a three-pronged moving body side section 132 that extends toward the distal end such that plate-shaped retaining plates 128 branch in three prongs so as to form gaps 130 having the same width as the gaps 108, and an insertion part 134, which extends toward the proximal end and joins with the proximal end side of the three-pronged moving body side section 132. Movable pin round holes 136 are formed on distal end parts of the three retaining plates 128. The movable pin 96 is inserted through the movable pin round holes 136, whereby attachment with the moving body 94 is completed. Further, a cylindrical body 138 is inserted in the insertion part 134 of the moving body 94. A retaining plate 128a, which extends in the center among the three retaining plates 128 of the moving body 94, is made of an insulating material.

The cylindrical body 138 extends a predetermined length (e.g., 6 mm) in the axial direction, and a hollow space 140 is formed in the interior of the cylindrical body 138. The insertion part 134 is inserted in a distal end portion of the cylindrical body 138, and a first connector 142 is inserted in a proximal end portion of the cylindrical body 138.

The first connector 142 includes a flange 144 disposed at an intermediate location, a distal end connecting projection 146 that extends toward the distal end from the flange 144, and a proximal end connecting projection 148 that extends toward the proximal end from the flange 144. The distal end connecting projection 146 of the first connector 142 is inserted into the cylindrical body 138, and the proximal end connecting projection 148 of the first connector 142 is inserted into the hollow tube 82, thereby connecting the cylindrical body 138 and the hollow tube 82 to each other. In a state in which the hollow tube 82 (first connector 142) is connected thereto, the cylindrical body 138 is inserted into the interior of the outer shell member 32.

The outer shell member 32 is made up from a distal end side tubular portion 150 in which the engagement tubular portion 114 of the gripper retaining member 30 is inserted, and a proximal end side tubular portion 152 in which the cylindrical body 138 is inserted. The distal end side tubular portion 150 includes an arcuate piece 150a that is separable in half from one side. On an inner surface of the distal end side tubular portion 150 (including the arcuate piece 150a), a groove 154 having a predetermined width (e.g., 2 mm) is provided. In the inserted condition of the gripper retaining member 30, the projection 120 thereof is inserted in engagement with the groove 154.

The cylindrical body 138, the first connector 142, and the distal end side of the hollow tube 82 are inserted into the proximal end side tubular portion 152. At upper and lower positions of the proximal end side tubular portion 152, outer shell member side hinge pieces 156 are formed, which project in the proximal end direction. The outer shell member side hinge pieces 156 are connected bendably to the bending portion 34 positioned on the proximal end side with respect to the hinge pieces 156. Further, cutout portions 158, which match substantially with the distal end shapes of the belts (first and second belts 66, 68), are formed as a pair on the outside surface of the proximal end side tubular portion 152. The distal ends of the belts are connected and fixed in the cutout portions 158 by fixing pins 160.

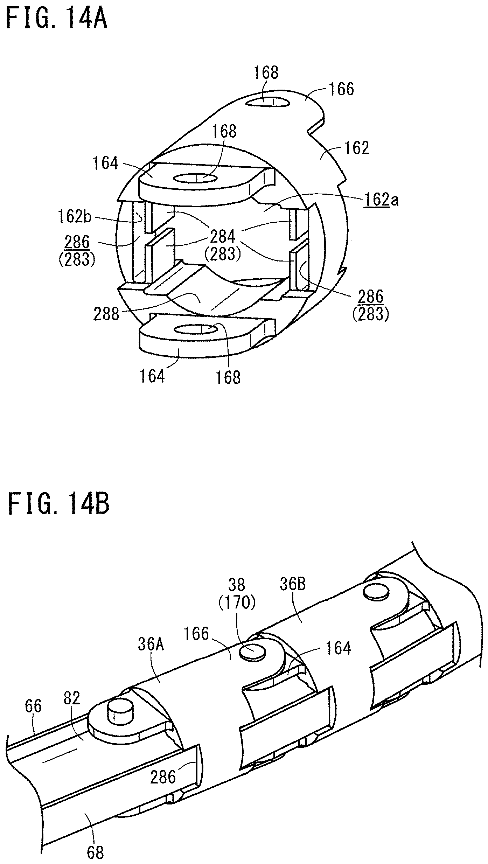

As shown in FIG. 8, the distal end working unit 14 includes a bending portion 34 that carries out the yawing operation of the gripper 12 (see FIG. 7) on a proximal end side with respect to the outer shell member 32. Four of the joint members 36A through 36D from among the five joint members 36A through 36E that make up the bending portion 34 are each equipped with a center tubular portion 162 (tubular portion), which is formed in a tubular shape in a central region thereof, distal end hinge pieces 164, which extend toward the distal end side from the center tubular portion 162, and proximal end hinge pieces 166, which extend toward the proximal end side from the center tubular portion 162. The center tubular portion 162 includes a hollow part 162a (see FIG. 14A) in a central portion thereof that penetrates in the axial direction.

The distal end hinge pieces 164 are formed to extend inwardly more than the proximal end hinge pieces 166. Each of the adjacent joint members 36 are connected such that the distal end hinge pieces 164 and the proximal end hinge pieces 166 overlap one another. Further, hinge holes 168 are formed in both the distal end hinge pieces 164 and the proximal end hinge pieces 166, and joint pins 170 are inserted in the hinge holes 168 in a state in which the distal end hinge pieces 164 and the proximal end hinge pieces 166 overlap. Consequently, the hinge members 38 are constructed such that the adjacent joint members 36 are mutually connected bendably about the joint pins 170. Further, the distal end hinge pieces 164 of the joint member 36A on the furthest most distal end side are connected in a bendable fashion to the outer shell member side hinge pieces 156 of the outer shell member 32.

On the other hand, although the joint member 36E on the furthest most proximal end side includes the center tubular portion 162 and the distal end hinge pieces 164 in the same manner as the joint members 36A through 36D, the joint member 36E is not provided with the proximal end hinge pieces 166 on the proximal end side of the center tubular portion 162, and includes a fitting extension 172, which is formed with an outer diameter matching substantially with the inner diameter of the outer tube 84. The fitting extension 172 of the joint member 36E is fitted into and fixed to the outer tube 84.

The flexible hollow tube 82 is inserted through the hollow parts 162a of the five joint members 36. The hollow tube 82 is formed with a predetermined inner diameter (e.g., 1.5 mm), and includes a hollow portion 82a that penetrates through the hollow tube 82 in the axial direction. The first connector 142 is connected to the distal end of the hollow tube 82, and a second connector 174 is connected to a proximal end of the hollow tube 82. The second connector 174, similar to the first connector 142, includes a flange 176 disposed at an intermediate location, a distal end connecting projection 178 that extends toward the distal end from the flange 176, and a proximal end connecting projection 180 that extends toward the proximal end from the flange 176. The distal end connecting projection 178 of the second connector 174 is inserted into the hollow tube 82, and the proximal end connecting projection 180 of the second connector 174 is inserted into the inner tube 86, thereby connecting the hollow tube 82 and the inner tube 86 to each other.

Further, the belts (the first and second belts 66, 68) are inserted through the bending portion 34 along inner side surfaces of the five joint members 36. Reinforcing plates 182 are bonded to inner sides of the first and second belts 66, 68, and the first and second belts 66, 68 extend in the axial direction. The distal ends of the first and second belts 66, 68 are connected to (the cutout portions 158 of) the outer shell member 32, whereas the proximal ends thereof are connected to the first and second semicircular pipes 70, 72 that are arranged between the outer tube 84 and the inner tube 86. The first and second belts 66, 68 are slidable with respect to the joint members 36, so that the outer shell member 32 on the distal end side can be tilted in lateral directions responsive to the sliding amount of the first and second belts 66, 68.

The outer tube 84 and the inner tube 86 that make up the shaft 16, and the first and second semicircular pipes 70, 72 that are disposed therebetween, extend in the proximal end direction and are inserted into the handle 18. As shown in FIG. 9, the handle main body 40 of the handle 18 is constructed so as to be capable of being separated (into a right side casing 40a and a left side casing 40b, see FIG. 1) in the vertical direction (the left side casing 40b is omitted from illustration in FIG. 9). Further, an insertion hole 42a for insertion therein of the shaft 16 is formed on the distal end surface of the handle main body 40.

A first bracket 200 to which the proximal end of the shaft 16 (outer tube 84) is connected, and a second bracket 202 that confronts the first bracket 200 are arranged in the interior space of the handle main body 40. The first bracket 200 is formed in a bent plate-like shape having a distal end surface and a right side surface, and the second bracket 202 is formed in a bent plate-like shape having an upper surface and a left side surface. A shaft insertion pipe 204, which extends in the distal end direction, is disposed on the distal end surface of the first bracket 200, and the shaft 16 is inserted into the shaft insertion pipe 204 in a condition in which the outer tube 84, the semicircular pipes, and the inner tube 86 overlap. Further, by insertion of the shaft insertion pipe 204 into a through hole 50a of the rotating handle 50, the rotating handle 50 is mounted rotatably with respect to the first bracket 200 (i.e., the handle 18).

The first and second slide members 74, 76 are arranged at positions near an upper portion between the first and second brackets 200, 202. The first and second slide members 74, 76 are substantially T-shaped as viewed from the side, and have bar portions 206 that extend in the distal end and proximal end directions, and attachment parts 208 that extend downwardly from the bar portions 206. The above-described racks 78 are disposed on confronting surfaces of the pair of bar portions 206. The bar portions 206 are disposed slidably in supporting cutouts 210 that are formed in the first bracket 200. A stopper 212, which regulates a movement limit of the proximal end side of the first and second slide members 74, 76, is disposed on the proximal end side of the first and second brackets 200, 202.

On the other hand, confronting surfaces on the lower end side of the pair of attachment parts 208 are formed in arcuate shapes, and are capable of coming into intimate contact with outer surfaces of the first and second semicircular pipes 70, 72. Stated otherwise, the first semicircular pipe 70 is connected to the attachment part 208 of the first slide member 74, and the second semicircular pipe 72 is connected to the attachment part 208 of the second slide member 76. Consequently, the proximal end sides of the first and second semicircular pipes 70, 72 are supported by the first and second slide members 74, 76, and the first and second semicircular pipes 70, 72 extend in the distal end direction between the outer tube 84 and the inner tube 86.

The pinion 80, which is disposed on the racks 78 between the first and second slide members 74, 76, is formed on the lower end of a pinion shaft 214. The pinion shaft 214 extends upwardly and is inserted through a pinion hole 202a provided in an upper surface of the second bracket 202. On the pinion shaft 214, a driven bevel gear 216 is fitted at a position located above the pinion 80. The driven bevel gear 216 is formed with a toothed surface on an upper side surface (inclined surface) thereof, and a toothed surface of a main drive bevel gear 218 is arranged in meshed engagement with respect to the toothed surface of the driven bevel gear 216. The main drive bevel gear 218 is connected to the distal end of a motor gear pin 220, and a motor-driven driven gear 222 is disposed on the proximal end of the motor gear pin 220.

In a state in which the drive unit 41 is attached to the handle main body 40, the motor-driven driven gear 222 is made to mesh with a motor-driven main drive gear 224 that projects from the distal end surface of the drive unit 41. The drive motor 46 (see FIG. 11) is arranged in the interior of the drive unit 41, and the motor-driven main drive gear 224 is driven rotatably by the drive motor 46. By such a structure, based on the rotary drive from the drive motor 46, the first and second slide members 74, 76 are made to move slidably in the distal end and proximal end directions, whereby the first and second semicircular pipes 70, 72 connected thereto are operated.

Further, the above-described switches 48 are disposed respectively on the right side surface of the first bracket 200 and on the left side surface of the second bracket 202. The switches 48 are exposed to the outside from windows 42b that are formed on side surfaces of the handle main body 40 (see FIG. 1). Non-illustrated electrical circuits, which are capable of sensing the operating state of the switches 48, are printed on outer side surfaces of the first and second brackets 200, 202 that are in contact with both ends of the switches 48. Further, a second signal connector (not shown) is disposed in the interior of the handle 18. The second signal connector is electrically connected to the aforementioned electrical circuits, and when the drive unit 41 is mounted, the second signal connector is connected to a first signal connector (not shown) of the drive unit 41. The manipulator 10 is constructed such that, by the connections of the first and second signal connectors, power is supplied to the electrical circuits, and operating signals (voltage values), which are generated by operating the switches 48, are transmitted to the external controller 20. Based on the operating signals, the controller 20 supplies power (signals) for rotating the drive motor 46.

Incidentally, the inner tube 86, which is covered by the first and second semicircular pipes 70, 72, extends in the proximal end direction more than the attachment parts 208 of the first and second slide members 74, 76. A rotary driven gear 226, which constitutes a portion of the rotation mechanism 88, and a sliding drive shaft 228 (rotating shaft member), which constitutes a portion of the advancing and retracting movement mechanism 98, are mounted on a proximal end side surface of the inner tube 86.

The rotary driven gear 226 extends a predetermined axial length (e.g., 6 mm), and a toothed surface, which is formed on the outer side surface thereof, meshes with a toothed surface of a rotary main drive gear 230, which is arranged on the lower side of the rotary driven gear 226. The rotary main drive gear 230 is connected and fixed to the proximal end of a rotating pin 232. The rotating pin 232 is axially supported rotatably by a retaining projection 234 that projects from the plate surface of the first bracket 200. Further, a handle-driven gear 236 is formed on the distal end of the rotating pin 232. The handle-driven gear 236 is arranged in front of the distal end surface of the first bracket 200. A toothed surface of the handle-driven gear 236 is enmeshed with a toothed surface of a handle main drive gear 238, which is disposed on the proximal end side of the rotating handle 50 and is arranged inside the body portion 42. By constructing the rotation mechanism 88 in this manner, the inner tube 86 is made to rotate about its axis based on a rotating operation of the rotating handle 50.

On the other hand, the sliding drive shaft 228 that is mounted on the inner tube 86 includes a head part 240 that is expanded radially outward on the distal end side, and a shank part 242 that extends a predetermined length toward the proximal end side from the head part 240. A recessed portion 244 (groove) is formed along the circumferential direction on an outer side surface of the head part 240, and projecting portions 248 (protrusion) of a responsive member 246 (moving body), which is arranged on the lower side of the sliding drive shaft 228, is inserted into the recessed portion 244.

The responsive member 246 is made up from a block body which is formed in an oval shape as viewed from the side, and a pair of arms 250 that extend from an upper portion of the block body. The projecting portions 248 are formed on inner side surfaces, respectively, of upper end sides of the pair of arms 250. Further, on a side surface of the responsive member 246, two holes (an upper side hole 246a and a lower side hole 246b) are formed. The upper side hole 246a is pivotally supported by a first support pin 252 that extends from the first bracket 200. Accordingly, the responsive member 246 is capable of rotating about the upper side hole 246a (first support pin 252). On the other hand, a spring link 256 is connected to the lower side hole 246b through a first link shaft 254.

The spring link 256 includes a zigzag-shaped spring member 258 in a central portion, together with two holes (front side hole 256a, rear side hole 256b) formed on both ends thereof. The aforementioned first link shaft 254 is inserted in the rear side hole 256b, and a second link shaft 260 is inserted in the front side hole 256a. The second link shaft 260 is connected to an upper end of the trigger 52.

The trigger 52 is constituted from an extending handle 262, which extends downwardly in a bifurcated form, and a linkage 264, which projects from an upper portion of the extending handle 262, and in which two holes (link hole 264a, support hole 264b) are formed to penetrate. The aforementioned second link shaft 260 is inserted into the link hole 264a. On the other hand, the support hole 264b is pivotally supported on a second support pin 266 that extends from the first bracket 200. Accordingly, the trigger 52 is capable of rotating about the support hole 264b (second support pin 266). By constructing the advancing and retracting movement mechanism 98 in this manner, the sliding drive shaft 228 is made to move in the distal end and proximal end directions, based on a pulling operation and a pushing operation of the trigger 52. As a result, advancing and retracting movements of the inner tube 86 can be implemented.

Further, a rotating part of a slip ring system 270 is disposed on the shank part 242 of the sliding drive shaft 228. The slip ring system 270 is a mechanism to continuously establish an electrical connection between the high frequency power source 22 and the conductive line 60 (the two metal members 56) arranged inside the inner tube 86, even while the inner tube 86 is being rotated by the rotation mechanism 88. More specifically, a first resin tube 272, a first cylindrical terminal 62a, a second resin tube 274, a second cylindrical terminal 62b, an insulating washer 276, and a nut 278 are mounted externally with respect to the shank part 242 of the sliding drive shaft 228. The first cylindrical terminal 62a is mounted on an outer side surface of the first resin tube 272, with one of the metal members 56 (e.g., a positive terminal) being connected to the interior thereof. The second cylindrical terminal 62b is mounted on an outer side surface of the second resin tube 274, with the other of the metal members 56 (e.g., a negative terminal) being connected to the interior thereof. The first and second contact terminals 64a, 64b abut respectively against side surfaces of the first and second cylindrical terminals 62a, 62b, and the first and second contact terminals 64a, 64b are connected to the high frequency power source 22. Consequently, in the slip ring system 270, the first and second contact terminals 64a, 64b continue to remain in abutment, even if the first and second cylindrical terminals 62a, 62b are rotating. Thus, the output from the high frequency power source 22 is supplied to the metal members 56.

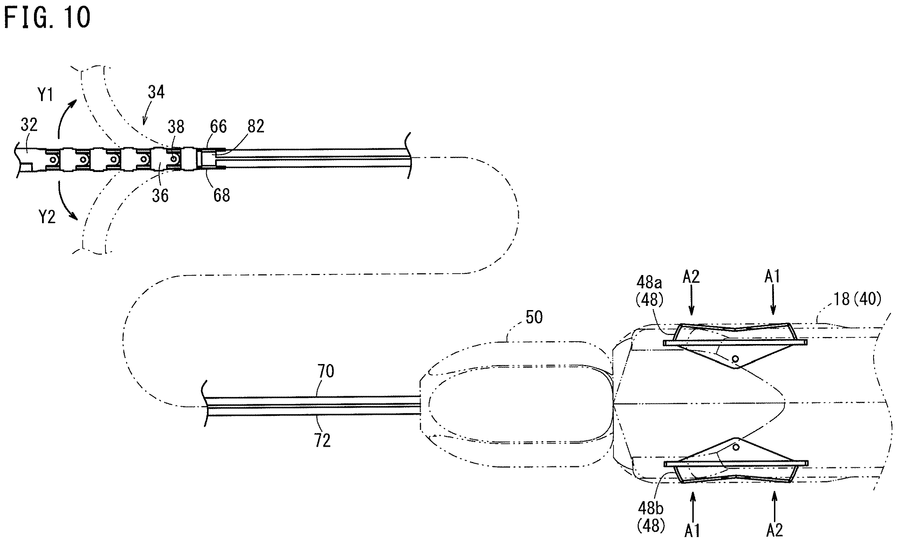

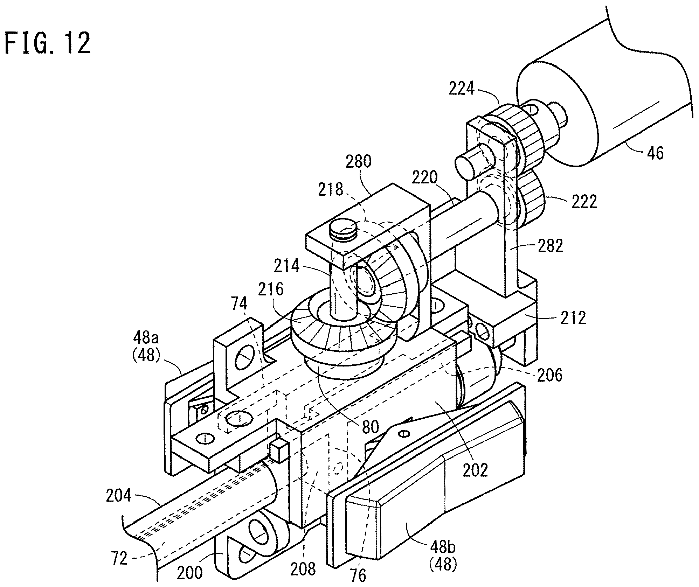

FIG. 10 is a schematic plan view for describing the yawing operation of the manipulator 10 of FIG. 1, FIG. 11 is a partial perspective view for describing the yawing operation of the manipulator 10 of FIG. 1, and FIG. 12 is a partial perspective view for describing an assembled condition in the interior of the handle 18 of FIG. 1.

Next, a structure in relation to the yawing operation of the manipulator 10 according to the present embodiment will be described in greater detail. As shown in FIGS. 9 and 10, on the handle 18, two switches 48 (right side switch 48a, left side switch 48b) are mounted on opposite side surfaces of the handle main body 40. Center portions of the right side and left side switches 48a, 48b are supported on the handle 18 (by the first and second brackets 200, 202), and a structure is provided such that both end portions (a distal end portion and a proximal end portion) thereof are displaced about the center portions in response to pushing operation of the switches by the user. The right side and left side switches 48a, 48b are structured to be capable of being handled by both left-handed and right-handed users. For example, in the case that the gripping unit 44 is gripped by the right hand, the right side switch 48a can be operated by the index finger of the right hand.

The right side switch 48a is constituted such that, in the case that the proximal end side (A1 in FIG. 10) thereof is pressed, the distal end working unit 14 undergoes a yawing operation in a rightward direction (Y1 direction), and in the case that the distal end side (A2 in FIG. 10) thereof is pressed, the distal end working unit 14 undergoes a yawing operation in a leftward direction (Y2 direction). On the other hand, the left side switch 48b is constituted such that, in the case that the proximal end side (A2 in FIG. 10) thereof is pressed, the distal end working unit 14 undergoes a yawing operation in a leftward direction (Y2 direction), and in the case that the distal end side (A1 in FIG. 10) thereof is pressed, the distal end working unit 14 undergoes a yawing operation in a rightward direction (Y1 direction). Owing to such a structure, whichever switch, i.e., the right side switch 48a or the left side switch 48b, the user operates, the feeling of operation of the switches 48 matches with the sense (viewpoint) of the user.

Stated otherwise, the operating signals that are generated by operating the switches 48 are of two patterns, i.e., a yawing operation signal in the left direction and a yawing operation signal in the right direction, and the signals are transmitted to the controller 20 (see FIG. 11). The controller 20 outputs, to the drive motor 46, a drive signal based on the operating signal for controlling the rotational speed, the rotational direction, etc. of the drive motor 46. Based on the drive signal from the controller 20, rotation of the drive motor 46 is carried out in the form of a clockwise rotation or a counterclockwise rotation. The driving control of the drive motor 46 may adopt various configurations. For example, configurations may be adopted in which, corresponding to the pressing force applied to the switches 48, the rotational speed of the drive motor 46 is varied, or the bending speed of the yawing operation is varied.

As shown in FIGS. 11 and 12, the manipulator 10 is constructed such that, by attaching the drive unit 41 to the handle main body 40, the motor-driven main drive gear 224 that is mounted on the distal end of the drive motor 46 meshes with the motor-driven driven gear 222 in the interior of the handle 18. The motor gear pin 220, which is equipped with the motor-driven driven gear 222, is retained rotatably by a first support bar 280 and a second support bar 282, which are erected upwardly from the proximal end side of the second bracket 202. The first support bar 280 includes an upper end portion which extends in the direction of the distal end and holds an upper end of the pinion shaft 214. The pinion shaft 214 is rotated by the driven bevel gear 216, which abuts against the main drive bevel gear 218 that is disposed on the distal end of the motor gear pin 220, so as to rotate the pinion 80 that is formed on the lower end of the driven bevel gear 216.

The pinion 80 is arranged in a space that is formed by assembly of the first and second brackets 200, 202. The first and second slide members 74, 76 are disposed in confronting relation sandwiching the pinion 80. The bar portions 206 of the first and second slide members 74, 76 are supported in the supporting cutouts 210 (see FIG. 9) of the first bracket 200. The first and second semicircular pipes 70, 72 are connected respectively to the attachment parts 208 that extend downwardly from the bar portions 206. Therefore, when the pinion 80 is rotated in one direction, the first slide member 74 and the second slide member 76 slide mutually in opposite directions, accompanied by the first semicircular pipe 70 and the second semicircular pipe 72 also sliding mutually in opposite directions. Sliding of the first and second semicircular pipes 70, 72 in opposite directions is transferred through the shaft 16 to the distal end working unit 14 on the distal end side.

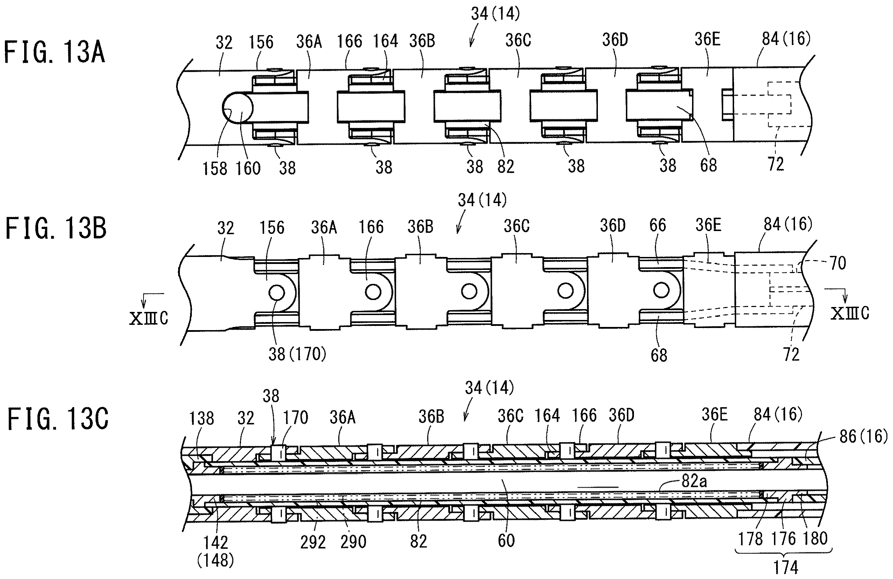

FIG. 13A is a side view showing main components of the bending portion 34 of the manipulator 10 of FIG. 1, FIG. 13B is a plan view showing main components of the bending portion 34 of the manipulator 10, and FIG. 13C is a cross sectional view taken along line XIIIC-XIIIC of FIG. 13B. FIG. 14A is a partial perspective view showing at an enlarged scale one of the joint members 36 of FIG. 13A, and FIG. 14B is a partial perspective view showing at an enlarged scale an assembled condition of the joint members 36.

As shown in FIGS. 13A and 13B, with the bending portion 34, the first and second belts 66, 68, which are connected to the distal ends of the first and second semicircular pipes 70, 72, are inserted through the five joint members 36, and are connected by fixing pins 160 to the outer shell member 32 on the distal end side. During the yawing operation, by sliding movement of the first and second belts 66, 68 in opposite directions, since one of the belts becomes shorter and the other of the belts becomes longer, the bending portion 34 is subjected to a bending operation. For implementing the bending operation, a structure is provided in which the first and second belts 66, 68 slide along inner side surfaces of the joint members 36.

As shown in FIG. 14A, guide members 283 that serve to guide the first and second belts 66, 68 are disposed in the center tubular portions 162 of the joint members 36 on an outer side of the hollow parts 162a of the center tubular portions 162. The guide members 283 comprise belt spaces 286 (penetrating cavities) that penetrate in the axial direction through the center tubular portion 162, and projecting walls 284 that act as separations between the hollow parts 162a and the belt spaces 286. The projecting walls 284 are erected in the hollow parts 162a perpendicularly to the distal end and proximal end hinge pieces 164, 166, which extend in pairs on upper and lower parts of the center tubular portion 162. The first and second belts 66, 68 are mounted so as to be inserted through the belt spaces 286 that are separated by the projecting walls 284 (see FIG. 14B).

On the other hand, the distal end hinge pieces 164 and the proximal end hinge pieces 166 are formed at positions shifted by 90° with respect to the belt spaces 286 through which the first and second belts 66, 68 are inserted. Accordingly, the distal end hinge pieces 164 and the proximal end hinge pieces 166 are bendably connected by the joint pins 170, and the adjacent joint members 36 are easily bent about the joint pins 170 responsive to sliding of the first and second belts 66, 68. Further, in the case that the first and second belts 66, 68 are slid (i.e., in the event that the bending portion 34 is subjected to bending), the first and second belts 66, 68 can avoid becoming separated from the inner side surfaces of the center tubular portions 162 by the projecting walls 284, and corresponding to the sliding amount, the joint members 36 can be bent together in conjunction.

Furthermore, arcuate surfaces 288 through which the hollow tube 82 can be inserted are formed on upper and lower inside surfaces of the joint members 36. In the event that the joint members 36 are bent, the hollow tube 82 bends in following relation thereto as a result of being guided by the arcuate surfaces 288.

The hollow tube 82 is flexible and is capable of being bent in following relation to the bending movement of the bending portion 34, and as shown in FIG. 13C, a hollow portion 82a is formed therein having an inner diameter that enables insertion of the conductive line 60. The second connector 174 is connected to the proximal end side of the hollow portion 82a, and the first connector 142 is connected to the distal end side of the hollow portion 82a.

Further, a double coil (a first coil 290, a second coil 292) is arranged in the interior of the hollow tube 82 to surround the hollow portion 82a. The first and second coils 290, 292 extend axially in an overlapping manner in a state of being wound mutually in different (clockwise, counterclockwise) winding directions. Consequently, when the hollow tube 82 is rotated clockwise, the coil (e.g., the first coil 290) that is wound in the clockwise direction expands in diameter, and the coil (e.g., the second coil 292) that is wound in the counterclockwise direction is reduced in diameter. Therefore, the shape of the double coil overall is maintained. As a result, even if the hollow tube 82 is rotated in a state in which the hollow tube 82 is bent by the bending portion 34, the rotational torque thereof can easily be transmitted to the distal end side (the gripper 12).

By constructing the manipulator 10 to include the distal end working unit 14 and the handle 18, as described above, the gripper 12 can be tilted in lateral directions by the yawing operation. For example, as shown in FIG. 11, in the event that the gripper 12 is to be tilted in a rightward direction, the user presses the proximal end A1 of the right side switch 48a (or the distal end A1 of the left side switch 48b) (see FIG. 10), whereby an operating signal from the switch 48 is sent to the controller 20. The controller 20 receives the operating signal and outputs, to the drive motor 46, a predetermined drive signal (electric power), and then the controller 20 rotates the motor-driven main drive gear 224 of the drive motor 46 in a clockwise direction. As a result, the motor gear pin 220 (and the motor-driven driven gear 222) is rotated in a counterclockwise direction, and the driven bevel gear 216 (and the pinion shaft 214), which is enmeshed with the main drive bevel gear 218, is rotated in a clockwise direction as viewed in plan. Accordingly, the pinion 80 is rotated clockwise, whereupon the first slide member 74 is made to slide in the proximal end direction, and the second slide member 76 is made to slide in the distal end direction.

Accompanying movement of the first slide member 74 in the proximal end direction, the first semicircular pipe 70 and the first belt 66 also undergo sliding movement in the proximal end direction. Further, accompanying movement of the second slide member 76 in the distal end direction, the second semicircular pipe 72 and the second belt 68 also undergo sliding movement in the distal end direction. Accordingly, in the bending portion 34, since the axial length of the first belt 66 becomes shorter, whereas the axial length of the second belt 68 becomes longer, the bending portion 34 undergoes a yawing operation and is bent on the side of the first belt 66. As a result, the gripper 12, which is disposed on the distal end side of the bending portion 34, is changed to a rightward tilted posture.

The bending portion 34 may be constructed such that, during bending of the bending portion 34, the sliding amounts of the first and second belts 66, 68 are changed responsive to the bending direction. More specifically, in the case that the bending portion 34 is bent, the length of the belt (first belt 66) on the inside of the bending direction becomes shorter than the belt on the outside (second belt 68) by an amount that is more than necessary. For this reason, a structure may be provided in which, by interposing non-illustrated gears between the pinion 80 and the racks 78 of the first and second slide members 74, 76 without the pinion 80 meshing directly with the racks 78, the sliding amount of the belt on the inside of the bending direction (i.e., the first belt 66 in the case of the right side, the second belt 68 in the case of the left side) can be reduced.

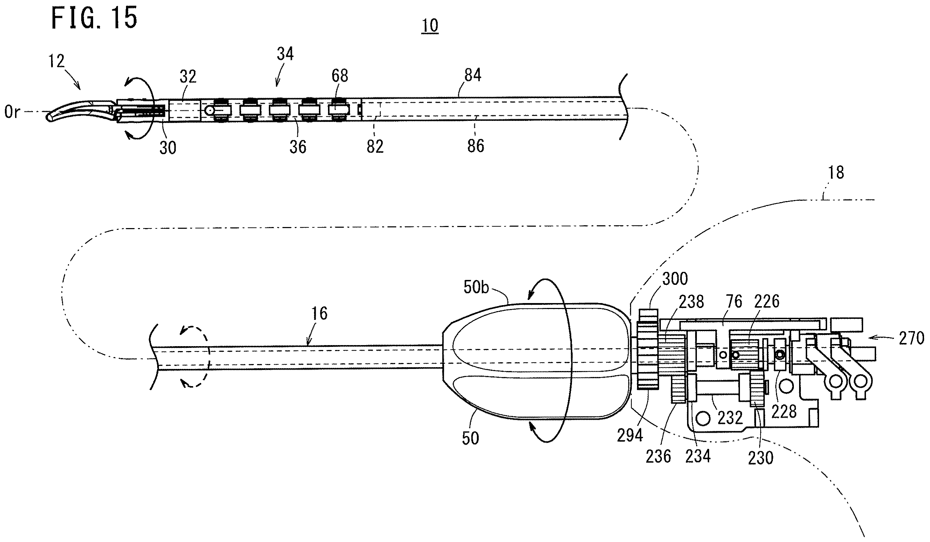

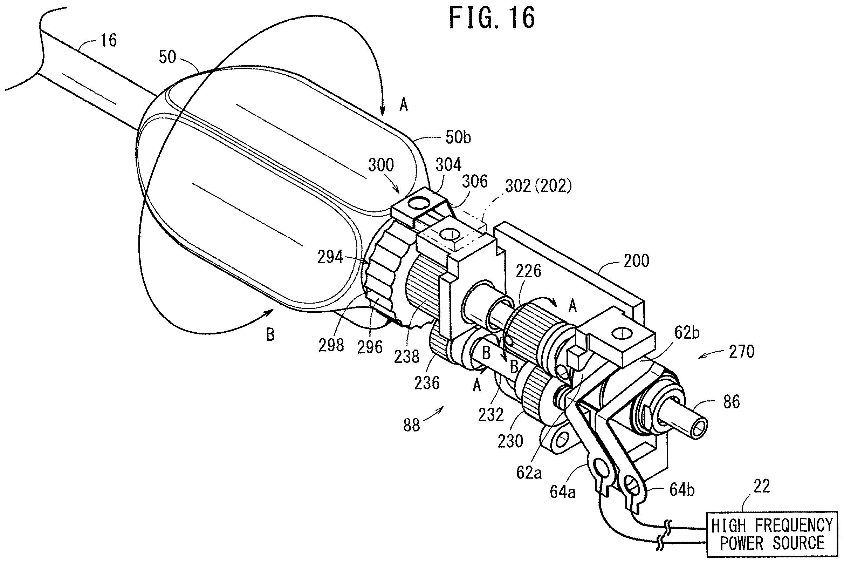

FIG. 15 is a schematic side view for describing the rolling operation of the manipulator 10 of FIG. 1, and FIG. 16 is a partial perspective view for describing an assembled condition in the interior of the handle 18 of FIG. 1.

Next, a structure in relation to the rolling operation of the manipulator 10 according to the present embodiment will be described in greater detail. As shown in FIG. 15, by the rolling operation, the gripper 12 on the distal end of the manipulator 10 is rotated about the axis Or of the gripper 12. In the present embodiment, the axis Or of the gripper 12 (end effector) is defined by the center axis of a portion that extends substantially in a straight line on the distal end side of the bending portion 34 (joint part). The axis Or is tilted in the yawing direction with respect to the axis Os of the shaft 16 (see FIG. 4B).

The rolling operation is implemented by the user manually operating the rotating handle 50 that is mounted on the distal end of the handle 18. The rotating handle 50 is a block shaped member having a width that is smaller than the width of the handle 18 as viewed in plan (see FIG. 10). The shaft insertion pipe 204 of the first bracket 200 is inserted into the through hole 50a that is provided in the center thereof (see FIG. 9). Further, plural blades 50b that make the rotating operation easier for the user are provided on side surfaces of the rotating handle 50.

As shown in FIGS. 15 and 16, a latch gear 294 and a handle main drive gear 238, which rotates together with the rotating handle 50, are disposed on the proximal end side of the rotating handle 50. The latch gear 294 and the handle main drive gear 238 are disposed in a distal end part of an interior space of the handle 18. The outer side surface of the latch gear 294 is formed in a wavelike shape (irregular surface) by arc-shaped valley portions 296, and mountain portions 298 that bulge upwardly between the valley portions 296. A latch piece 300 is configured to abut against the outer side surface of the latch gear 294.

The latch piece 300 includes a mounting portion 304, which is attached to a mounting plate 302 that extends in the distal end direction from the second bracket 202, and an elastic piece 306 that extends obliquely downward from the mounting portion 304 and is capable of swinging elastically with respect to the mounting portion 304 (see FIG. 9). The lower end of the elastic piece 306 is formed in an arcuate shape that matches with the valley portions 296 of the latch gear 294, and in a mounted state of the latch piece 300, is in surface contact with the valley portions 296. When the rotating handle 50 is rotated, the latch gear 294 also rotates, and by such rotation, the latch piece 300 (the elastic piece 306) swings elastically along the valley portions 296 and the mountain portions 298. When the elastic piece 306 overcomes the mountain portions 298 and moves into the valley portions 296, a positive displacement takes place due to the elastic force thereof, and the displacement of the latch piece 300 is transmitted as a tactile sensation to the user that operates the rotating handle 50. Furthermore, along with displacement of the latch piece 300, since operating noise is generated, the operational feeling of the rotating handle 50 can be recognized more strongly by the user. Consequently, the user can perform the rotating operation of the rotating handle 50 more intuitively.

The handle main drive gear 238 rotates the rotating pin 232 (handle-driven gear 236) that is supported on the first bracket 200. The handle-driven gear 236 is formed with a smaller outer diameter than the handle main drive gear 238, and is rotated at a greater amount of rotation than the amount of rotation of the rotating handle 50. The rotary main drive gear 230, which is disposed on the proximal end side of the rotating pin 232, rotates the rotary driven gear 226 that is fixed to the inner tube 86 of the shaft 16, and the rotary driven gear 226 is rotated integrally with the inner tube 86.

Further, the sliding drive shaft 228 and the slip ring system 270 (excluding the first and second contact terminals 64a, 64b), which are mounted more on the proximal end side than the rotary driven gear 226, rotate together with the inner tube 86. The first and second contact terminals 64a, 64b abut elastically against the first and second cylindrical terminals 62a, 62b, so as to remain in contact therewith even though the first and second cylindrical terminals 62a, 62b are rotating. Thus, steady electrical conduction is realized between the high frequency power source 22 and the conductive line 60 that is arranged in the interior of the inner tube 86.

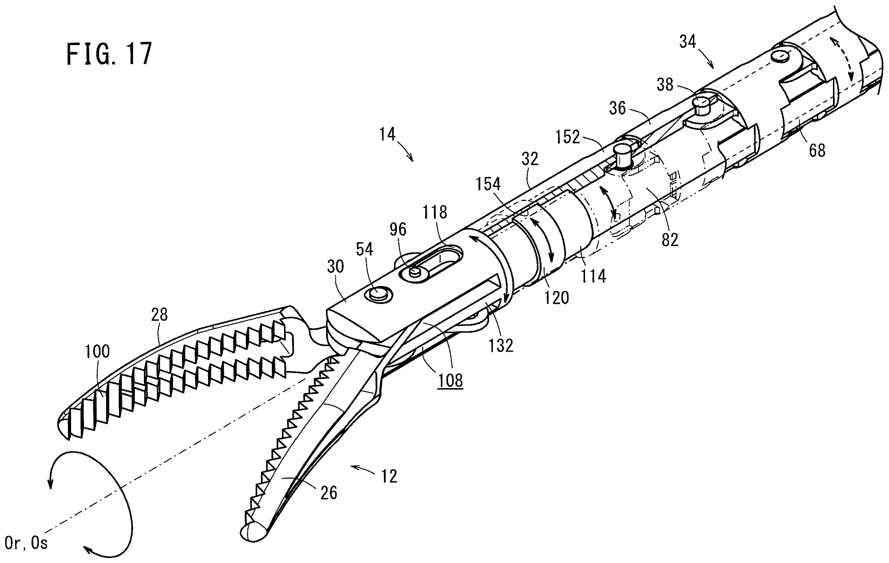

FIG. 17 is a perspective view, partially cut away, of main components of a distal end part of the manipulator 10 of FIG. 1, FIG. 18A is a cross sectional plan view showing a distal end part of the manipulator 10 of FIG. 1, and FIG. 18B is a cross sectional side view showing a distal end part of the manipulator 10 of FIG. 1.

As shown in FIG. 17, the distal end working unit 14 is constituted such that the gripper 12 rotates in the circumferential direction on the distal end side of the bending portion 34. More specifically, by connecting the outer shell member 32 to the distal end of the joint members 36 that constitute the bending portion 34, a structure is provided in which the outer shell member 32 does not rotate. On the other hand, by transferring the rotational torque from the hollow tube 82 that is disposed in the interior of the bending portion 34, a structure is provided in which the gripper 12 and the gripper retaining member 30 are rotated in the circumferential direction about the axis of the gripper 12.

More specifically, the hollow tube 82 is connected through the second connector 174 to the inner tube 86 of the shaft 16, whereby the inner tube 86 rotates together with the hollow tube 82 (see FIG. 13C). As shown in FIGS. 18A and 18B, the distal end of the hollow tube 82 is inserted into the engagement tubular portion 114 of the gripper retaining member 30, and is fitted and connected to the proximal end connecting projection 148 of the first connector 142. Further, the distal end connecting projection 146 of the first connector 142 is fitted into the cylindrical body 138.

The cylindrical body 138 is formed with an outer diameter that matches substantially with the inner diameter of the engagement tubular portion 114. To the engagement tubular portion 114, the rotational torque is transferred from the cylindrical body 138 that is connected to the hollow tube 82. More specifically, when the hollow tube 82 is rotated and the rotational torque therefrom is transmitted, the first connector 142 and the cylindrical body 138 rotate together integrally, and the gripper retaining member 30 (the engagement tubular portion 114) is rotated accompanying rotation of the cylindrical body 138.

The insertion part 134 of the aforementioned moving body 94 is inserted in a distal end portion of the cylindrical body 138. The retaining plate 128a at the center of the moving body 94 is made of an insulating material, and the retaining plate 128a extends to the proximal end side of the insertion part 134, with welded portions 308 being provided on both upper and lower surfaces of the proximal end part thereof. The metal members 56 (metal wires), which are exposed from the distal end of the conductive line 60, are welded by a welding material to the welded portions 308. The conductive line 60 extends in the axial direction in the hollow portion 82a of the hollow tube 82 while a distal end part thereof is fixedly retained by the first connector 142. By fitting the insertion part 134 in the cylindrical body 138, the moving body 94 rotates along with the rotation of the cylindrical body 138. Also, by transmitting rotation of the moving body 94 to the gripper retaining member 30, the gripper retaining member 30 is rotated more smoothly.

Because the first and second gripper members 26, 28 that make up the gripper 12 are supported by the gripper retaining member 30, the first and second gripper members 26, 28 rotate together with rotation of the gripper retaining member 30. More specifically, in the manipulator 10, as shown in FIG. 17, the gripper 12, the gripper retaining member 30, and the hollow tube 82 are rotated about the axis Or of the gripper 12.

In particular, in the manipulator 10 according to the present embodiment, the rolling operation of the gripper 12 enables the gripper 12 to be rotated through a 360° unlimited range of rotation with respect to the axis Or of the gripper 12. More specifically, the inner tube 86 is rotatable in an unlimited manner with respect to the outer tube 84, and the hollow tube 82, which is arranged at a position overlapping with the bending portion 34, also is rotatable in an unlimited manner. Similarly, the gripper retaining member 30 (gripper 12), which is inserted in the outer shell member 32, is rotatable in an unlimited manner. Further, since the conductive line 60 extends inside the hollow tube 82 and the inner tube 86, the conductive line 60 rotates together with the hollow tube 82 and the inner tube 86. At this time, conduction of electricity with the high frequency power source 22 at the proximal end side of the conductive line 60 is continued by the slip ring system 270.

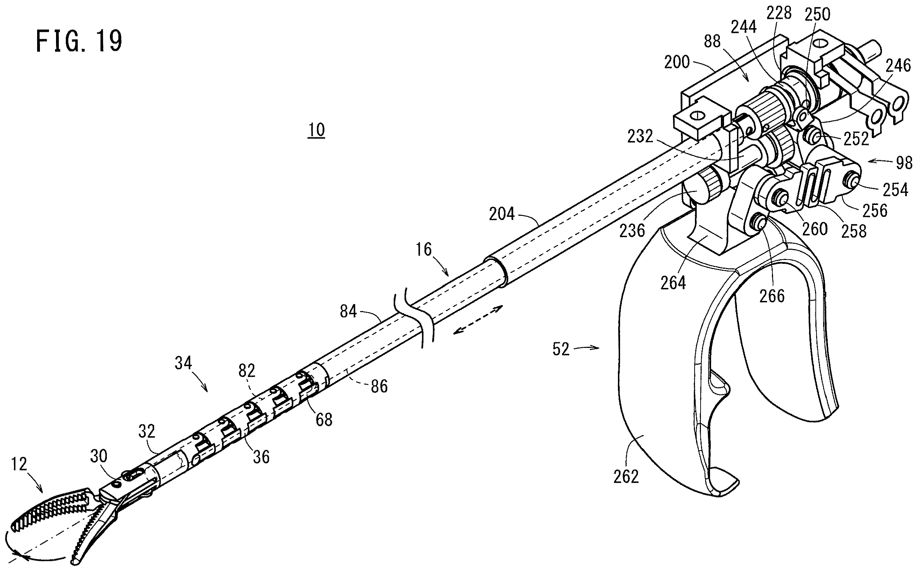

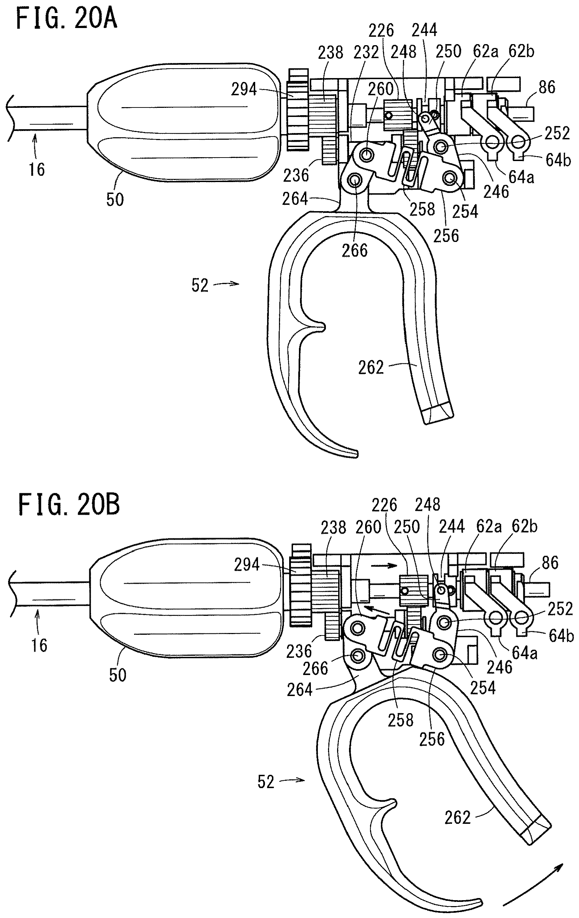

FIG. 19 is a schematic perspective view for describing the opening and closing operation of the gripper 12 of FIG. 1, FIG. 20A is a first side view for describing operations on the side of the handle 18 of FIG. 19, and FIG. 20B is a second side view for describing operations on the side of the handle 18 of FIG. 19.

Next, a structure in relation to the opening and closing operation of the gripper 12 of the manipulator 10 according to the present embodiment will be described in greater detail. As shown in FIG. 19, by the opening and closing operation, distal end portions (serrated meshing teeth 100) of the first and second gripper members 26, 28 of the gripper 12 are made to approach and separate away from each other. The opening and closing operation is implemented by the user manually operating the trigger 52 of the handle 18.

As shown in FIGS. 20A and 20B, the trigger 52 is arranged on the lower side of the rotating pin 232 that makes up the rotation mechanism 88, and is supported swingably with respect to the first bracket 200 (handle 18). More specifically, the linkage 264 of the trigger 52 is supported by the second support pin 266 that extends from the first bracket 200, and the extending handle 262 is operated in the distal end and proximal end directions about the second support pin 266.

The linkage 264 of the trigger 52 is connected to the advancing and retracting movement mechanism 98 that operates the inner tube 86. More specifically, a distal end of the spring link 256 is connected through the second link shaft 260 to an upper portion (link hole 264a) above the insertion position of the second support pin 266 of the linkage 264. The spring link 256 is arranged to extend in the proximal end direction, and the proximal end thereof is connected through the first link shaft 254 to a lower end portion (lower side hole 246b) of the responsive member 246. The fist support pin 252 that extends from the first bracket 200 is inserted into the upper side hole 246a of the responsive member 246, and the projecting portion 248 (see FIG. 9) of the pair of arms 250 that extend upwardly above the upper side hole 246a is inserted into the recessed portion 244 of the sliding drive shaft 228.

Consequently, when the extending handle 262 of the trigger 52 is pulled in the proximal end direction, the trigger 52 rotates about the second support pin 266, and the upper end (the connection location of the spring link 256) of the linkage 264 is moved in the direction of the distal end. Along therewith, the spring link 256 also moves in the distal end direction, whereby the lower end of the responsive member 246 is made to move in the distal end direction. By the lower end moving in the distal end direction, the responsive member 246 rotates about the first support pin 252, and the arms 250 on the upper end side are made to move in the direction of the proximal end. Accordingly, the sliding drive shaft 228 (recessed portion 244) is pressed by the projecting portion 248 and moves in the direction of the proximal end. As a result, the inner tube 86 moves in the proximal end direction together with the sliding drive shaft 228, and the operating force in the proximal end direction is transferred through the shaft 16 to the distal end working unit 14 on the distal end side.

Moreover, in the event that the extending handle 262 of the trigger 52 is operated by being pulled, the spring member 258 of the spring link 256 expands elastically, and serves to suppress movement of the inner tube 86 in the proximal end direction. Stated otherwise, the movement amount when the inner tube 86 is moved in the proximal end direction is limited to the degree at which the gripper 12 shifts to the closed state from the open state, and in the case that the trigger 52 is pulled beyond the above degree, the operating force is absorbed by elastic deformation of the spring member 258. As a result, application of a large torque during the closing operation of the gripper 12 can be avoided. For example, movement of the inner tube 86 in the proximal end direction is of a degree that enables transitioning from the condition shown in FIG. 20A to the condition shown in FIG. 20B.

The axial length of the rotary driven gear 226 and the two cylindrical terminals 62, which are mounted on the inner tube 86, is set to be longer than the movement amount in advancing and retracting directions of the inner tube 86. Therefore, even in the case that the trigger 52 is operated and the inner tube 86 is moved in the proximal end direction, the rotary main drive gear 230 can mesh continuously with the rotary driven gear 226, and the first and second contact terminals 64a, 64b can remain in contact continuously with the first and second cylindrical terminals 62a, 62b. Consequently, while the inner tube 86 undergoes rotation (i.e., while the rolling operation of the gripper 12 is carried out), opening and closing of the gripper 12 can be performed, and current can be supplied to energize the treatment target X.

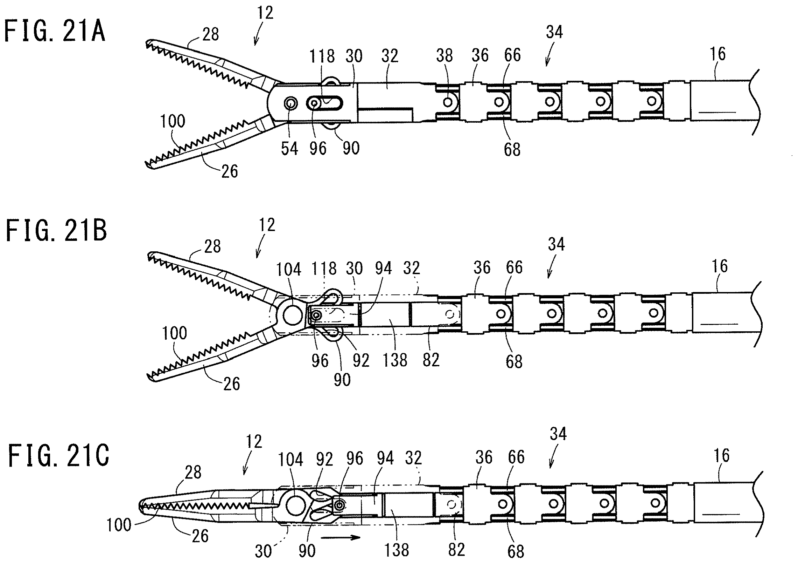

FIG. 21A is a principal plan view showing the gripper 12 of FIG. 1, FIG. 21B is a partial plan view showing an open state of the gripper 12, and FIG. 21C is a partial plan view showing a closed state of the gripper 12. The operating force that moves the inner tube 86 in the distal end and proximal end directions is transmitted to the hollow tube 82, which is connected to the distal end of the inner tube 86, whereby the hollow tube 82 is made to move in the distal end and proximal and directions. The cylindrical body 138 is connected through the first connector 142 to the distal end of the hollow tube 82, and the cylindrical body 138 also is made to move together with the hollow tube 82.

The length in the axial direction of the hollow tube 82 is longer than the axial length of the bending portion 34 of the distal end working unit 14, such that even if the hollow tube 82 is advanced and retracted, the hollow tube 82 is arranged to overlap with the bending portion 34 at all times. Accordingly, even if the bending portion 34 is subjected to bending as shown in FIG. 11, and the hollow tube 82 bends in following relation thereto, the hollow tube 82 can be advanced and retracted along the bend, and the operating force of the advancing and retracting movement can be transmitted to the cylindrical body 138 that is connected to the distal end side of the hollow tube 82.

As shown in FIG. 18A, connecting pins 310a, 310b, which project inwardly (into the hollow space 140) by a predetermined amount, are disposed on the distal end side and the proximal end side of the cylindrical body 138. The first connector 142 is equipped with a first connector side engagement groove 312, which is engraved in a circumferential direction in a root portion of the distal end connecting projection 146. The proximal end side connecting pin 310a is inserted into the first connector side engagement groove 312. Further, the moving body 94 is equipped with a moving body side engagement groove 314, which is engraved in a circumferential direction in the insertion part 134 that projects in the proximal end direction. The distal end side connecting pin 310b is inserted into the moving body side engagement groove 314. By this structure, the axial connections between the first connector 142, the cylindrical body 138, and the moving body 94 are reinforced by the connecting pins 310a, 310b, and the operating force of the advancing and retracting movement, which is transmitted from the hollow tube 82, is further transmitted reliably to the moving body through the cylindrical body 138.

On the other hand, the gripper retaining member 30 is placed in a condition in which the moving body 94 is arranged in the sliding space 122 thereof, and the gripper retaining member 30 remains fixed with respect to movements of the moving body 94 in the distal end and proximal end directions. More specifically, by engagement between the projection 120, which is formed on the outer side surface of the engagement tubular portion 114, and the groove 154, which is formed on the inner side surface of the outer shell member 32, the gripper retaining member 30 is placed in a fixed state in which back and forth movement thereof is restricted, in contrast to the advancing and retracting movements of the moving body 94.

In the moving body 94, the first and second gripper members 26, 28 are sandwiched between the gaps 130 of (the three-pronged moving body side section 132 of) the distal end, and the movable pin 96, which is inserted in the long holes 92 of the extension pieces 90, is retained therein. As shown in FIGS. 21A through 21C, in a condition in which the moving body 94 is positioned on the distal end side of the sliding space 122, the movable pin 96 is moved to the distal end side of the long holes 92, and the distal end portions of the first and second gripper members 26, 28 are separated and placed in an open condition.

With respect to the open condition, when the inner tube 86 is moved in the proximal end direction by a pulling operation of the trigger 52, an operating force in the proximal end direction is transmitted to the moving body 94 via the inner tube 86, the second connector 174, the hollow tube 82, the first connector 142, and the cylindrical body 138. At this time, the first connector 142, the cylindrical body 138, and the moving body 94 are connected by the connecting pins 310a, 310b, whereby the operating force in the proximal end direction is transferred smoothly. When the moving body 94 is moved in the proximal end direction, the movable pin 96 moves in the proximal end direction while guiding the long holes 92, and based on the guiding of the long holes 92, the distal end parts of the first and second gripper members 26, 28 are made to approach one another mutually, and a closed condition can be obtained.

Further, as discussed above, in the close state of the gripper 12, a current supplying condition is formed by the connection of the first and second gripper members 26, 28, which are of different polarities. More specifically, in a state in which the treatment target X is sandwiched between the first and second gripper members 26, 28, an output, which is supplied to the first and second gripper members 26, 28 through the conductive line 60 from the high frequency power source 22, is supplied to the treatment target X, and a predetermined process (cauterization by application of heat, or the like) is performed thereon.

In the foregoing manner, by means of the manipulator 10 according to the present embodiment, the hollow tube 82 that is disposed in a position overlapping with the bending portion 34 bends in following relation with the yawing operation of the bending portion 34. Therefore, for example, even in the case that the treatment target X is located deeply inside the living body, the posture (inclination) of the gripper 12 can easily be changed, and the gripper 12 can be delivered smoothly to the treatment target X.

Further, in the distal end working unit 14, an operating force in the direction of rotation is transmitted to the gripper 12 by the hollow tube 82, whereby the gripper 12 is rotated (subjected to a rolling operation) through an unlimited range of rotation. Therefore, the posture of the gripper 12 (i.e., a posture in the direction of rotation around the roll axis Or of the gripper 12) on the distal end side of the bending portion 34 can be changed freely, and the orientation of the gripper 12 can be changed any number of times to match with the treatment target X, and to enable an accurate treatment to be carried out on the treatment target X.

Further, by constructing the bending portion 34 from the plural (five) joint members 36, the bending portion 34 can be bent gently and gradually. Therefore, the hollow tube 82, which is disposed at a position overlapping with the bending portion 34, can easily follow the bending movement of the bending portion 34. In addition, since the hollow tube 82 extends a greater length than the bending portion 34, the operating force in the direction of rotation and the operating force in the distal end and proximal end directions, which are transmitted from the inner tube 86, can be transferred smoothly beyond the bending portion 34 to the distal end side (gripper 12).

Furthermore, in accordance with the operating forces in the distal end and proximal end directions that are transmitted through the hollow tube 82, the opening and closing operation of the gripper 12 can easily be carried out. Consequently, the treatment target X can easily be gripped by the gripper 12, and a predetermined procedure (energizing treatment) can be performed efficiently on the treatment target X. The operating forces in the distal end and proximal end directions may be used not only for carrying out an opening and closing operation of the gripper 12, but also for carrying out, for example, an operation for moving, in the distal end and proximal end directions, the end effector as it is.

Still further, by rotating the hollow tube 82 in a position overlapping with the bending portion 34, and thereby rotating the gripper retaining member 30 that is connected to the hollow tube 82 relatively with respect to the outer shell member 32, the gripper 12 that is held by the gripper retaining member 30 can be rotated suitably. At this time, the projection 120 of the gripper retaining member 30 is inserted rotatably in the groove 154 of the outer shell member 32, whereby rotation of the gripper retaining member 30 is guided by the groove 154. Thus, the gripper 12 can be rotated more smoothly, and the gripper 12 can be oriented in a desired posture.

Further still, by arranging the first and second coils 290, 292, which are wound in different winding directions, in the interior (hollow portion 82a) of the hollow tube 82, the strength of the hollow tube 82 can be increased. More specifically, the rotational operating forces that are transmitted from the inner tube 86 can be transferred smoothly to the gripper 12 through the hollow tube 82, the strength of which is increased and the shape of which is maintained.

Further, by means of the manipulator 10 according to the present embodiment, even if the hollow tube 82, which is disposed in a position overlapping with the bending portion 34, is subjected to bending by the bending portion 34, the operating force from the inner tube 86 is transferred to the distal end side (the gripper retaining member 30 and the moving body 94) relative to the bending portion 34, while the hollow tube 82 remains in a bent condition. Thus, at the distal end side with respect to the bending portion 34, variations in posture or opening and closing operations of the gripper 12 can easily be performed.

In this case, by disposing the conductive line 60 in the hollow portion 82a of the hollow tube 82, a conductive path leading to the gripper 12, which is constituted as a bipolar type of electrosurgical knife, can easily be constructed, and electrical power can be supplied stably to the gripper 12.

For example, in the event that the operating force transmitted from the handle 18 is an operating force for rotating the inner tube 86, the hollow tube 82 is rotated by the operating force, and the gripper 12, which is connected to the distal end side of the hollow tube 82, is made to undergo a rolling operation, whereby the gripper 12 can be made to face toward the treatment target X at a desired posture. At this time, by integral rotation of the conductive line 60 that is accommodated in the hollow tube 82, disconnection between the gripper 12 and the conductive line 60 (conductive path) can reliably be prevented.

Further, as described above, the slip ring system 270 is provided in the manipulator 10. Specifically, a structure is enabled in which the contact terminals 64 are electrically connected to the cylindrical terminals 62 during times that the cylindrical terminals 62 are rotating and at rest. Consequently, even if the inner tube 86 is rotated in order for the gripper 12 to undergo the rolling operation, conduction of electricity with the high frequency power source 22 can continuously be maintained. Accordingly, even if the posture of the gripper 12 in the direction of rotation is changed, an electrical treatment can suitably be carried out.

Further, for example, in the event that the operating force transmitted from the handle 18 is an operating force for moving the inner tube 86 in the distal end and proximal end directions, the hollow tube 82 is advanced and retracted together with movement of the inner tube 86, whereby the gripper 12 can be opened and closed. At this time, by integral advancing and retracting movement of the conductive line 60 integrally with the hollow tube 82, disconnection between the gripper 12 and the conductive line 60 (conductive path) can reliably be prevented.

Furthermore, by means of the manipulator 10 according to the present embodiment, by constructing the bending portion 34 from the plural (five) joint members 36, the hinge members 38, and the first and second belts 66, 68, the gripper 12 can easily be bent in a direction that differs from the axis Os of the shaft 16. Further, through application of the first and second belts 66, 68, the alignment of the plural joint members 36 can stably be maintained. For example, an operation to restore the bending portion 34 to its origin (where the bending portion 34 is aligned with the axial direction of the shaft 16) or the like can be performed quickly and accurately. Furthermore, in the case that the first and second belts 66, 68 cause the bending portion 34 to bend, the plural joint members 36 can smoothly be bent together in conjunction. The belts are not limited to the structure described above in which a pair (two) of such belts are disposed on the bending portion 34, and one or three or more of such belts may be provided.

In the case that a pair of belts (the first and second belts 66, 68) are provided, by relative operations of the first and second belts 66, 68, the bending portion 34 can be bent smoothly. In other words, by carrying out an operation to move the first belt 66 in the proximal end direction and to move the second belt 68 oppositely in the distal end direction, the gripper 12 can easily be tilted to the side of the first belt 66.

Further, by providing the pinion 80 and the first and second slide members 74, 76 in the handle 18, the rotational driving force of the pinion 80 can easily be converted into operating forces in the distal end and proximal end directions of the first and second slide members 74, 76. In addition, the operating forces can be transmitted easily to the first and second belts 66, 68 through the first and second semicircular pipes 70, 72. In this case, by controlling the rotational amount of the pinion 80, the amount by which the bending portion 34 curves (i.e., the tilt angle of the gripper 12) can be changed freely.

Furthermore, in the manipulator 10, by the first and second belts 66, 68 being guided by the guide members 283, the first and second belts 66, 68 move smoothly with respect to the center tubular portion 162, and the operating force that moves the first and second belts 66, 68 in the distal end and proximal end directions can easily be transferred to the surrounding joint members 36. At the time that the bending portion 34 is bent, outward bulging of the first and second belts 66, 68 can be suppressed. Furthermore, by disposing the guide members 283 on the outer side of the hollow parts 162a, various transmission members that serve to operate the end effector (gripper 12) can appropriately be arranged in the hollow parts 162a.

Further, the guide members 283 are equipped with the projecting walls 284 that function as separations between the hollow parts 162a and the belt spaces 286. Thus, since flexure of the first and second belts 66, 68 toward the inside (toward the centers of the joint members 36) is restricted by the projecting walls 284 at the time that the bending portion 34 is bent, the bending portion 34 can be bent more smoothly.

Furthermore, by forming the arcuate surfaces 288 that are capable of contacting the hollow tube 82 on the inner side surface of the center tubular portions 162, in the case that the bending portion 34 is bent, the hollow tube 82 that is inserted through the interior of the center tubular portions 162 can also easily be bent.

Still further, with the manipulator 10 according to the present embodiment, by providing the rotation mechanism 88 and the advancing and retracting movement mechanism 98 in the interior of the handle 18, the inner tube 86 can be moved in the distal end and proximal end directions, and the inner tube 86 can be rotated. Two types of operating forces of the inner tube 86 are transmitted to the gripper 12 of the distal end working unit 14 that is disposed on the distal end of the shaft 16, whereby variations in posture and opening and closing operations of the gripper 12 can be carried out easily.

In this case, by forming the length in the axial direction of at least one of the rotary driven gear 226 and the rotary main drive gear 230 of the rotation mechanism 88 to be longer than the movement amount of the inner tube 86 performed by the advancing and retracting movement mechanism 98, even if the inner tube 86 is moved in the distal end and proximal end directions by the advancing and retracting movement mechanism 98, the rotary driven gear 226 and the rotary main drive gear 230 can be kept in meshing engagement with each other at all times. Thus, the inner tube 86 can be rotated in a stable fashion.

Further, by disposing the rotating handle 50 at a position in the vicinity of the handle 18, in a state in which the user of the manipulator 10 has gripped the handle 18, the user can easily manipulate the rotating handle 50, which is in such a nearby position. Further, by forming the rotating handle 50 to be narrower than the width of the handle 18, in the case that multiple medical devices (a forceps, a manipulator 10, etc.) are used during a procedure, interference of the rotating handle 50 with such medical devices can be reduced.

Furthermore, by providing the latch gear 294 that rotates accompanying rotation of the rotating handle 50 and the latch piece 300 that abuts elastically against the latch gear 294, when the rotating handle 50 is operated, a latching sensation can be imparted to the user. Consequently, the user can recognize intuitively the amount of rotation of the rotating handle 50, and can easily adjust the rotation of the inner tube 86.

Still further, in the advancing and retracting movement mechanism 98, based on rotational operations of the responsive member 246, the projecting portion 248 is displaced in the distal end and proximal end directions, and the sliding drive shaft 228 including the recessed portion 244 in which the projecting portion 248 is inserted also is displaced in the distal end and proximal end directions. Accordingly, the inner tube 86 on which the sliding drive shaft 228 is installed can move smoothly in the distal end and proximal end directions. Further, since the recessed portion 244 is formed in the circumferential direction, the sliding drive shaft 228 can rotate relatively with respect to the projecting portion 248, and with a simple structure, rotation of the inner tube 86 can be allowed.

More specifically, with the manipulator 10 according to the present embodiment, high operability of the gripper 12 that is inserted in the interior of the living body can be obtained, and the procedure performed on the treatment target X can be conducted efficiently and accurately.

Although a certain preferred embodiment of the present invention has been shown and described in detail above, it should be understood that the present invention is not limited to the above embodiment and various changes and modifications may be made to the embodiment without departing from the scope of the invention as set forth in the appended claims. For example, in the above-described manipulator 10, a structure is provided in which only the yawing operation is made to operate electrically (through the drive motor 46). However, the invention is not limited to this feature. More specifically, in the manipulator 10, among the yawing operation, the rolling operation, and the opening and closing operation, any one or two or more of these operations can be constituted as an electrically driven operation, or alternatively the yawing operation, the rolling operation, and the opening and closing operation can all be constituted as manually driven operations.

## Claims

1. A medical manipulator (10) comprising:
a shaft (16);
a distal end working unit (14) provided on a distal end of the shaft (16) and having an end effector (12) configured to carry out a treatment on a treatment target; and
a main body portion (18) disposed on a proximal end of the shaft (16) and configured to operate the end effector (12) based on an input from a person,
wherein the distal end working unit (14) comprises:
a bending portion (34) disposed between the end effector (12) and the shaft (16) and which causes the distal end working unit (14) to bend in a direction that differs from an axial direction of the shaft (16), in accordance with an operating force in a bending direction that is transmitted from the main body portion (18); and
a tube (82), which is disposed at a position overlapping with the bending portion (34) and is capable of bending in following relation to movement of the bending portion (34), wherein the tube (82) transmits a rotational operating force, which is transmitted from the main body portion (18), to the end effector (12), so as to enable the end effector (12) to be rotated through an unlimited range of rotation, **characterized in that** the distal end working unit (14) transmits operating forces in a distal end direction and a proximal end direction, which are transferred from the main body portion (18), to the end effector (12) through the tube (82).

2. The medical manipulator (10) according to claim 1, wherein:
the bending portion (34) comprises a structure in which three or more rigid joint members (36) are arrayed in the axial direction, and the adjacent joint members (36) are connected to each other so as to bend mutually; and
the tube (82) extends over a length that is longer than an axial length of the bending portion (34).

3. The medical manipulator (10) according to claim 1, wherein:
the end effector (12) is a gripper (12) in which first and second gripper members (26, 28) are opened and closed to grip the treatment target; and
the gripper (12) closes the first and second gripper members (26, 28) by the operating force in the proximal end direction that is transmitted through the tube (82), and opens the first and second gripper members (26, 28) by the operating force in the distal end direction that is transmitted through the tube (82).

4. The medical manipulator (10) according to claim 1, wherein the distal end working unit (14) comprises:
an outer shell member (32) connected to a distal end of the bending portion (34); and
a retaining member (30), which retains the end effector (12) under a condition in which at least a portion of the retaining member (30) is accommodated in an interior of the outer shell member (32), and is rotated relatively with respect to the outer shell member (32) by the rotational operating force from the tube (82).

5. The medical manipulator (10) according to claim 4, wherein:
the outer shell member (32) has a groove (154) that is engraved in a circumferential direction on an inner wall confronting an outer surface of the retaining member (30); and
the retaining member (30) has a projection (120) that is inserted rotatably in the groove (154).

6. The medical manipulator (10) according to claim 1, wherein:
plural coils (290, 292) are disposed in an interior of the tube (82) in an overlapping manner so as to surround a hollow portion (82a) that extends in the axial direction; and
at least two coils among the plural coils (290, 292) are wound in mutually different winding directions.

## Patentansprüche

1. Medizinischer Manipulator (10), mit:
einem Schaft (16);
einer distalendigen Arbeitseinheit (14), die an einem distalen Ende des Schaftes (16) bereitgestellt ist und einen Endeffektor (12) aufweist, der dazu ausgebildet ist, eine Behandlung an einem Behandlungsziel auszuführen; und
einem Hauptkörperabschnitt (18), der an einem proximalen Ende des Schaftes (16) angeordnet und dazu ausgebildet ist, den Endeffektor (12) basierend auf einer Eingabe von einer Person zu betätigen,
wobei die distalendige Arbeitseinheit (14) aufweist:
einen Abwinkelabschnitt (34), der zwischen dem Endeffektor (12) und dem Schaft (16) angeordnet ist und der die distalendige Arbeitseinheit (14) veranlasst, in einer Richtung abzuwinkeln, die sich von einer axialen Richtung des Schaftes (16) unterscheidet, in Übereinstimmung mit einer Betätigungskraft in einer Abwinkelrichtung, die von dem Hauptkörperabschnitt (18) übertragen wird; und
ein Rohr (82), das an einer Position angeordnet ist, die mit dem Abwinkelabschnitt (34) überlappt und sich in einer Folgebeziehung zur Bewegung des Abwinkelabschnitts (34) abwinkeln kann, wobei das Rohr (82) eine Drehbetätigungskraft überträgt, die von dem Hauptkörperabschnitt (18) zu dem Endeffektor (12) übertragen wird, um es so dem Endeffektor (12) zu ermöglichen, durch einen unbegrenzten Drehbereich gedreht zu werden, **dadurch gekennzeichnet, dass** die distalendige Arbeitseinheit (14) Betätigungskräfte in einer distalendigen Richtung und einer proximalendigen Richtung überträgt, die von dem Hauptkörperabschnitt (18) zu dem Endeffektor (12) durch das Rohr (82) übertragen werden.

2. Medizinischer Manipulator (10) nach Anspruch 1, wobei:
der Abwinkelabschnitt (34) eine Struktur aufweist, in der drei oder mehr starre Gelenkelemente (36) in der axialen Richtung in Reihe angeordnet sind, und die benachbarten Gelenkelemente (36) miteinander verbunden sind, um sich so wechselseitig abzuwinkein; und
das Rohr (82) sich über eine Länge erstreckt, die länger ist als eine axiale Länge des Abwinkelabschnitts (34).

3. Medizinischer Manipulator (10) nach Anspruch 1, wobei:
der Endeffektor (12) ein Greifer (12) ist, bei dem ein erstes und zweites Greifelement (26, 28) geöffnet und geschlossen werden, um das Behandlungsziel zu greifen; und
der Greifer (12) das erste und zweite Greifelement (26, 28) durch die Betätigungskraft in der proximalendigen Richtung, die durch das Rohr (82) übertragen wird, schließt und das erste und zweite Greifelement (26, 28) durch die Betätigungskraft in der distalendigen Richtung, die durch das Rohr (82) übertragen wird, öffnet.

4. Medizinischer Manipulator (10) nach Anspruch 1, wobei die distalendige Arbeitseinheit (14) aufweist:
ein äußeres Schalenelement (32), das mit einem distalen Ende des Abwinkelabschnitts (34) verbunden ist; und
ein Halteelement (30), das den Endeffektor (12) in einem Zustand hält, in dem zumindest ein Abschnitt des Halteelements (30) in einen Innenraum des äußeren Schalenelements (32) aufgenommen ist, und relativ zu dem äußeren Schalenelement (32) durch die Drehbetätigungskraft von dem Rohr (82) gedreht wird.

5. Medizinischer Manipulator (10) nach Anspruch 4, wobei:
das äußere Schalenelement (32) eine Nut (154) aufweist, die in einer Umfangsrichtung an einer Innenwand eingebracht ist, die einer äußeren Fläche des Halteelements (30) gegenüberliegt; und
das Halteelement (30) einen Vorsprung (120) aufweist, der drehbar in die Nut (154) eingesetzt ist.

6. Medizinischer Manipulator (10) nach Anspruch 1, wobei:
mehrere Spulen (290, 292) in einem Innenraum des Rohrs (82) in überlappender Weise angeordnet sind, um so einen hohlen Abschnitt (82a) zu umgeben, der sich in der axialen Richtung erstreckt; und
zumindest zwei Spulen unter den mehreren Spulen (290, 292) in wechselseitig unterschiedlichen Windungsrichtungen gewunden sind.

## Revendications

1. Manipulateur médical (10) comprenant :
un arbre (16) ;
une unité de travail d'extrémité distale (14) prévue sur une extrémité distale de l'arbre (16) et ayant un organe terminal effecteur (12) configuré pour réaliser un traitement sur une cible de traitement ; et
une partie de corps principal (18) disposée sur une extrémité proximale de l'arbre (16) et configurée pour faire fonctionner l'organe terminal effecteur (12) sur la base d'une entrée provenant d'une personne,
dans lequel l'unité de travail d'extrémité distale (14) comprend :
une partie de pliage (34) disposée entre l'organe terminal effecteur (12) et l'arbre (16) et qui provoque le pliage de l'unité de travail d'extrémité distale (14) dans une direction qui est différente d'une direction axiale de l'arbre (16), conformément à une force d'actionnement dans une direction de pliage qui est transmise depuis la partie de corps principal (18) ; et
un tube (82), qui est disposé à une position se chevauchant avec la partie de pliage (34) et est capable de se plier en suivant le mouvement de la partie de pliage (34), où le tube (82) transmet une force d'actionnement en rotation, qui est transmise à partir de la partie de corps principal (18), à l'organe terminal effecteur (12), de manière à permettre à l'organe terminal effecteur (12) de tourner selon une plage de rotation illimitée, **caractérisé en ce que** l'unité de travail d'extrémité distale (14) transmet des forces d'actionnement dans une direction d'extrémité distale et une direction d'extrémité proximale, qui sont transférées depuis la partie de corps principal (18) vers l'organe terminal effecteur (12) à travers le tube (82) .

2. Manipulateur médical (10) selon la revendication 1, dans lequel :
la partie de pliage (34) comprend une structure dans laquelle trois éléments de jonction (36) ou plus sont disposés en réseau dans la direction axiale, et les éléments de jonction adjacents (36) sont reliés l'un à l'autre de manière à se plier mutuellement ; et
le tube (82) s'étend sur une longueur qui est supérieure à une longueur axiale de la partie de pliage (34).

3. Manipulateur médical (10) selon la revendication 1, dans lequel :
l'organe terminal effecteur (12) est une pince (12) dans laquelle des premier et deuxième éléments de pince (26, 28) sont ouverts et fermés pour saisir la cible de traitement ; et
la pince (12) ferme les premier et deuxième éléments de pince (26, 28) par la force d'actionnement dans la direction d'extrémité proximale qui est transmise à travers le tube (82), et ouvre les premier et deuxième éléments de pince (26, 28) par la force d'actionnement dans la direction d'extrémité distale qui est transmise à travers le tube (82).

4. Manipulateur médical (10) selon la revendication 1, dans lequel l'unité de travail d'extrémité distale (14) comprend :
un élément de coque externe (32) relié à une extrémité distale de la partie de pliage (34) ; et
un élément de retenue (30), qui retient l'organe terminal effecteur (12) dans une condition dans laquelle au moins une partie de l'élément de retenue (30) est logée dans l'intérieur de l'élément de coque externe (32) et est tournée relativement par rapport à l'élément de coque externe (32) par la force d'actionnement en rotation provenant du tube (82).

5. Manipulateur médical (10) selon la revendication 4, dans lequel :
l'élément de coque externe (32) a une rainure (154) qui est gravée dans une direction circonférentielle sur une paroi interne faisant face à une surface externe de l'élément de retenue (30) ; et
l'élément de retenue (30) présente une saillie (120) qui est insérée en rotation dans la rainure (154).

6. Manipulateur médical (10) selon la revendication 1, dans lequel :
plusieurs bobines (290, 292) sont disposées dans un intérieur du tube (82) de manière chevauchante de façon à entourer une partie creuse (82a) qui s'étend dans la direction axiale ; et
au moins deux bobines parmi la pluralité de bobines (290, 292) sont enroulées dans des directions d'enroulement mutuellement différentes.
